# EUROPEAN PATENT SPECIFICATION

(11) **EP 2 209 831 B1**
(45) Date of publication and mention of the grant of the patent: **11.06.2014**
(21) Application number: 08845756.9
(22) Date of filing: 23.10.2008
(51) Int. Cl.: C08G 63/08, C08L 67/04, A61L 27/18, A61L 31/06

(54) **IMPLANTABLE DEVICES PROVIDING CONTROLLED RELEASE OF HYDROPHOBIC DRUGS**
IMPLANTIERBAREN VORRICHTUNGEN, DIE EINE KONTROLLIERTE FREISETZUNG VON HYDROPHOBEN ARZNEISTOFFEN ERMÖGLICHEN
DISPOSITIFS IMPLANTABLES ASSURANT UNE LIBÉRATION CONTRÔLÉE DE MÉDICAMENTS HYDROPHOBES

(30) Priority: 31.10.2007 US 982160
(43) Date of publication of application: 28.07.2010
(73) Proprietor: Abbott Cardiovascular Systems Inc., Santa Clara, CA 95054-2807 (US)
(72) Inventor: LIM, Florencia, Union City CA 94587 (US); NGO, Michael, Huy, San Jose CA 95128 (US); TANG, Yiwen, San Jose CA 95117 (US); HOSSAINY, Syed, F. A., Hayward CA 94544 (US); TROLLSAS, Mikael, O., San Jose CA 95124 (US)
(74) Representative: ZBM Patents - Zea, Barlocci & Markvardsen
(86) International application number: PCT/US2008/081016
(87) International publication number: WO 2009/058666

(56) References cited:
- EP-A- 0 420 541
- EP-A- 0 761 712
- EP-A- 1 121 943
- WO-A-03/020330
- WO-A-2004/108111
- WO-A-2008/058660
- JP-A- 2005 230 211
- US-A1- 2003 139 567

## Description

### BACKGROUND

### Field of the Invention

The present invention is directed to an implantable device comprising a coating comprising biodegradable polymeric materials providing controlled release of hydrophobic drugs.

### Description of the State of the Art

Angioplasty is a well-known procedure for treating heart disease. A problem associated with angioplasty includes formation of intimal flaps or torn arterial linings which can collapse and occlude the conduit after the balloon is deflated. Moreover, thrombosis and restenosis of the artery may develop over several months after angioplasty, which may require another angioplasty procedure or a surgical by-pass operation. "Stenosis" refers to a narrowing or constriction of the diameter of a bodily passage or orifice, and "restenosis" refers to the reoccurrence of stenosis in a blood vessel or heart valve after it has been treated (as by balloon angioplasty, stenting, or valvuloplasty) with apparent success.

Stents are often used in the treatment of atherosclerotic stenoses in blood vessels. To reduce the partial or total occlusion of the artery by the collapse of arterial lining and to reduce the chance of thrombosis and restenosis following angioplasty in the vascular system, a stent can be implanted in the lumen to reinforce body vessels and maintain the vascular patency. A "lumen" refers to a cavity of a tubular organ such as a blood vessel. As a mechanical intervention, stents act as scaffoldings, functioning to physically hold open and, if desired, to expand the wall of a passageway, e.g., a blood vessel, urinary tract or bile duct.

Stents are also used as a vehicle for providing biological therapy. Biological therapy can be achieved by medicating the stents. Medicated stents provide for the local administration of a therapeutic substance at the treatment site, thereby possibly avoiding side effects associated with systemic administration of such substance. One method of medicating stents involves the use of a polymeric carrier coated over the surface of a stent, wherein a therapeutic substance is impregnated in the polymeric carrier.

Late stent thrombosis has emerged as a concern for drug-delivery stents. The incidence of late stent thrombosis appears to be higher with drug-delivery stents than with the corresponding bare metal stents. One potential cause of late thrombosis with drug-delivery stents is a chronic inflammatory or hypersensitivity response to the polymeric coating over the stent.

To reduce the risk of late stent thrombosis, the stent can be coated with a biodegradable polymer derived from one or more hydrophilic monomers. At typical drug-to-polymer mass ratios, however, many hydrophobic drugs exhibit unsatisfactory release profiles with conventional biodegradable polymers. By contrast, the present invention provides biodegradable polymeric materials that release hydrophobic drugs from implantable devices in a controlled manner.

Document WO 2004/108111 discloses a depot composition, for example in the form of gels, for sustained delivery of a beneficial agent. The composition comprises a biodegradable polymer derived from lactide, glycolide and caprolactone. Document WO 2004/108111 faces the control of the drug release from implantable depot compositions that are applied to a desired side of action, in particular tissues of the body.

### SUMMARY OF THE INVENTION

The present invention is directed to an implantable device (e.g. stents) comprising a coating comprising biodegradable polymeric materials that provide controlled release of hydrophobic drugs as defined in the claims. The polymeric materials are derived from two or more relatively polar monomers so as to completely or substantially completely erode after the devices accomplish their intended functions (e.g., maintaining vascular patency and locally delivering drugs), thereby avoiding adverse effects such as late stent thrombosis. Further, the polymeric materials are derived from one or more relatively nonpolar monomers that increase the miscibility of hydrophobic drugs with the polymer and enhance the polymer's permeability to hydrophobic drugs. Other advantages of the biodegradable polymeric materials include, e.g., good mechanical properties (e.g., toughness and flexibility) and good physical properties (e.g., degradation rate and drug-release rate).

The invention relates to an implantable device comprising a coating comprising a composition, wherein the coating has a thickness of ≤ about 6 micron and completely degrades, which is the lost of at least about 95 % of its mass, or substantially completely degrades, which is the lost of at least 75% of its mass, within about 12 months when exposed to physiological conditions,
wherein the composition comprises a biodegradable block copolymer, wherein the copolymer:
is derived from at least two polar monomers selected from glycolide (GA), D-lactide (DLA), L-lactide (LLA), D,L-lactide (DLLA), and meso-lactide (MLA) and at least one nonpolar monomer selected from valerolactone (VL), caprolactone (CL), trimethylene carbonate (TMC), dioxanone (DS), hydroxybutyrate (HB), and hydroxyvalerate (HV);
has a T_{g} from about -150 °C to about 100 °C;
has a polymer number-average molecular weight (Mₙ) from about 10 kDa to
   about 500 kDa; and
completely degrades, which is the lost of at least about 95 % of its mass, or substantially completely degrades, which is the lost of at least 75% of its mass, within about 12 months;
   and wherein:
each kind of monomer independently has from about 10 to about 5,000 units
   in the copolymer; and
the molar % of each kind of monomer independently is from about 5% to about 90%.

In one embodiment, the biodegradable copolymer is selected from P(DLA-GA-VL), P(DLA-GA-CL), P(DLA-GA-TMC), P(DLA-GA-DS), P(DLA-GA-HB), P(DLA-GA-HV), P(LLA-GA-VL), P(LLA-GA-CL), P(LLA-GA-TMC), P(LLA-GADS), P(LLA-GA-HB), P(LLA-GA-HV), P(DLLA-GA-VL), P(DLLA-GA-CL), P(DLLA-GA-TMC), P(DLLA-GA-DS), P(DLLA-GA-HB), P(DLLA-GA-HV), P(MLA-GA-VL), P(MLA-GA-CL), P(MLA-GA-TMC), P(MLA-GA-DS), P(MLA-GA-HB), and P(MLA-GA-HV).

In some embodiments, the biodegradable copolymer has a degree of crystallinity of less than 50% or less than 20%. In certain embodiments, the copolymer is amorphous.

In further embodiments, the composition additionally comprises one or more components selected from biocompatible moieties, non-fouling moieties, biobeneficial materials, and biologically active agents, where the bioactive agents have a sustained release up to 12 months. In certain embodiments, the bioactive agents are hydrophobic drugs, e.g., rapamycin and derivatives thereof.

In an embodiment, the material is a coating disposed over at least a portion of the implantable device.

In certain embodiments, the implantable device is selected from stents, grafts, stent-grafts, catheters, leads, electrodes, clips, shunts, closure devices, valves, and particles. In a particular embodiment, the implantable device is a stent.

In one embodiment, the material is a coating disposed over at least a portion of the implantable device. In a specific embodiment, the implantable device is a stent.

Various embodiments of the invention are described in further detail below.

### BRIEF DESCRIPTION OF THE DRAWINGS

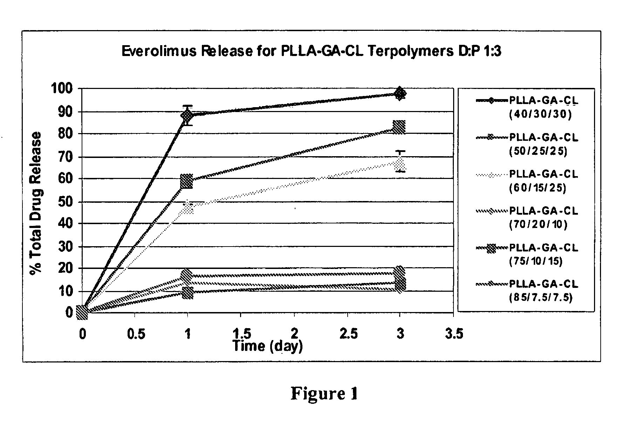

**Figure 1** shows the release rates of everolimus from stents coated with P(LLA-GA-CL) terpolymers containing various molar percentages of LLA, GA and CL, where the D:P ratio was 1:3 and the dosage of everolimus was about 100 microgram/cm².

### DETAILED DESCRIPTION OF THE INVENTION

### Terms and Definitions

The following definitions apply:

The terms "biologically degradable" (or "biodegradable"), "biologically erodable" (or "bioerodable"), "biologically absorbable" (or "bioabsorbable"), and "biologically resorbable" (or "bioresorbable"), in reference to polymers and coatings, are used interchangeably and refer to polymers and coatings that are capable of being completely or substantially completely degraded, dissolved, and/or eroded over time when exposed to physiological conditions and can be gradually resorbed, absorbed and/or eliminated by the body, or that can be degraded into fragments that can pass through the kidney membrane of an animal (e.g., a human), e.g., fragments having a molecular weight of about 40,000 Daltons (40 kDa) or less. The process of breaking down and eventual absorption and elimination of the polymer or coating can be caused by, e.g., hydrolysis, metabolic processes, oxidation, enzymatic processes, bulk or surface erosion, and the like. Conversely, a "biostable" polymer or coating refers to a polymer or coating that is not biodegradable.

Whenever reference is made to "biologically degradable," "biologically erodable," "biologically absorbable," or "biologically resorbable" stent coatings or polymers forming such stent coatings, it is understood that after the process of degradation, erosion, absorption, and/or resorption has been completed or substantially completed, no coating or substantially little coating will remain on the stent.

"Complete degradation" of a polymer or a polymeric material (e.g., a polymeric coating) means that the polymer or the polymeric material loses at least about 95% of its mass over a period of time.

"Substantially complete degradation" of a polymer or a polymeric material (e.g., a polymeric coating) means that the polymer or the polymeric material loses at least about 75% of its mass over a period of time. In certain embodiments, "substantially complete degradation" of a polymer or a polymeric material can mean that the polymer or the polymeric material loses at least about 80% of its mass, or at least about 85% of its mass, or at least about 90% of its mass, or at least about 95% of its mass over a period of time.

As used herein, a "biocompatible moiety" refers to a moiety that is capable of enhancing the biological compatibility of the composition, material (e.g., coating) or structure (e.g., implantable device) containing it by not causing injury or toxicity to, or an immunological reaction in, living tissue.

A "biobeneficial material" refers to a material that benefits a treatment site (e.g., by enhancing the biocompatibility of the implantable device containing such material) by being, e.g., non-fouling, hemocompatible, non-thrombogenic, and/or anti-inflammatory without depending on the release of a pharmaceutically or therapeutically active agent.

A "non-fouling moiety" is a moiety that provides an implantable device fabricated from or coated with a material comprising the moiety with the ability to resist (i.e., to prevent, delay, or reduce the amount of) build-up of a denatured layer of protein on its surface, which is caused by the body's reaction to foreign material and could lead to protein fouling. The adsorption of proteins on the surface of an implanted device constitutes the first step of several biological responses, including the activation of the coagulation cascade. Following protein adsorption, cell adhesion occurs, which could lead to impairment of the device's functioning as well as adverse side effects on the patient. For example, thrombi formation could occur after adsorption and activation of platelets.

"Physiological conditions" refer to conditions to which an implant is exposed within the body of an animal (e.g., a human). Physiological conditions include, but are not limited to, "normal" body temperature for that species of animal (approximately 37 °C for a human) and an aqueous environment of physiologic ionic strength, pH and enzymes. In some cases, the body temperature of a particular animal may be above or below what would be considered "normal" body temperature for that species of animal. For example, the body temperature of a human may be above or below approximately 37 °C in certain cases. The scope of the present invention encompasses those cases where the physiological conditions (e.g., body temperature) of an animal are not considered "normal".

In the context of a blood-contacting implantable device, a "prohealing" drug or agent refers to a drug or agent that has the property that it promotes or enhances re-endothelialization of arterial lumen to promote healing of the vascular tissue.

As used herein, a "co-drug" is a drug that is administered concurrently or sequentially with another drug to achieve a particular pharmacological effect. The effect may be general or specific. The co-drug may exert an effect different from that of the other drug, or it may promote, enhance or potentiate the effect of the other drug.

As used herein, the term "prodrug" refers to an agent rendered less active by a chemical or biological moiety, which metabolizes into or undergoes *in vivo* hydrolysis to form a drug or an active ingredient thereof. The term "prodrug" can be used interchangeably with terms such as "proagent", "latentiated drugs", "bioreversible derivatives", and "congeners". N.J. Harper, Drug latentiation, Prog Drug Res., 4: 221-294 (1962); E.B. Roche, Design of Biopharmaceutical Properties through Prodrugs and Analogs, Washington, DC: American Pharmaceutical Association (1977); A.A. Sinkula and S.H. Yalkowsky, Rationale for design of biologically reversible drug derivatives: prodrugs, J. Pharm. Sci., 64: 181-210 (1975). Use of the term "prodrug" usually implies a covalent link between a drug and a chemical moiety, though some authors also use it to characterize some forms of salts of the active drug molecule. Although there is no strict universal definition of a prodrug itself, and the definition may vary from author to author, prodrugs can generally be defined as pharmacologically inactive or less active chemical derivatives that can be converted *in vivo,* enzymatically or nonenzymatically, to the active, or more active, drug molecules that exert a therapeutic, prophylactic or diagnostic effect. Sinkula and Yalkowsky, above; V.J. Stella et al., Prodrugs: Do they have advantages in clinical practice?, Drugs, 29: 455-473 (1985).

The terms "polymer" and "polymeric" refer to compounds that are the product of a polymerization reaction. These terms are inclusive of homopolymers (i.e., polymers obtained by polymerizing one kind of monomer), copolymers (i.e., polymers obtained by polymerizing two or more different kinds of monomers), terpolymers (i.e., polymers obtained by polymerizing three different kinds of monomers), etc., including random, alternating, block, graft, star, dendritic, crosslinked and any other variations thereof.

The terms "block copolymer" and "graft copolymer" are defined in accordance with the terminology used by the International Union of Pure and Applied Chemistry (IUPAC). "Block copolymer" refers to a copolymer containing a linear arrangement of blocks. The block is defined as a portion of a polymer molecule in which the monomer units have at least one constitutional or configurational feature absent from the adjacent portions. "Graft copolymer" refers to a polymer composed of macromolecules with one or more species of block connected to the main chain as side chains, these side chains having constitutional or configurational features that differ from those in the main chain.

As used herein, a "polar monomer" is a monomer that is more polar than a "nonpolar monomer". Likewise, a "nonpolar monomer" is a monomer that is less polar than a "polar monomer". The terms "polar monomer" and "relatively polar monomer" are used interchangeably herein, and the terms "nonpolar monomer" and "relatively nonpolar monomer" are also used interchangeably herein. In some embodiments, a "polar monomer" is a monomer that is more hydrophilic than a "nonpolar monomer". Likewise, in some embodiments a "nonpolar monomer" is a monomer that is more hydrophobic than a "polar monomer".

As used herein, an "implantable device" can be any suitable substrate that can be implanted in a human or non-human animal. Examples of implantable devices include, but are not limited to, self-expandable stents, balloon-expandable stents, coronary stents, peripheral stents, stent-grafts, catheters, other expandable tubular devices for various bodily lumen or orifices, grafts, vascular grafts, arterio-venous grafts, by-pass grafts, pacemakers and defibrillators, leads and electrodes for the preceding, artificial heart valves, anastomotic clips, arterial closure devices, patent foramen ovale closure devices, cerebrospinal fluid shunts, and particles (e.g., drug-delivery particles, microparticles and nanoparticles). The stents can be intended for any vessel in the body, including neurological, carotid, vein graft, coronary, aortic, renal, iliac, femoral, popliteal vasculature, and urethral passages.

An implantable device can be designed for the localized delivery of a therapeutic agent. A medicated implantable device can be constructed in part, e.g., by coating the device with a coating material containing a therapeutic agent. The body of the device can also contain a therapeutic agent.

An implantable device can be coated with a coating containing partially or completely a biodegradable/bioabsorbable/bioerodable polymer, a biostable polymer, or a combination thereof. A portion of the implantable device or the whole device itself can also be fabricated partially or completely from a biodegradable/bioabsorbable/bioerodable polymer, a biostable polymer, or a combination thereof.

As used herein, a "portion" of an implantable device can be any portion of the device. For example, a portion can be a portion of the body of the device. As another example, a portion can be a portion of the surface of the device, or the whole surface of the device. As a further example, a portion can refer to an area of material in the body or over the surface of the device, e.g., a layer, film or coating disposed over the device.

As used herein, a material (e.g., a layer, film or coating) "disposed over" a substrate (e.g., an implantable device) is deposited directly or indirectly over at least a portion of the surface of the substrate. Direct depositing means that the material is applied directly to the exposed surface of the substrate. Indirect depositing means that the material is applied to an intervening material that has been deposited directly or indirectly over the substrate.

The "glass transition temperature", T_{g}, is the temperature at which the amorphous domains of a polymer change from a solid glassy state to a solid deformable or rubbery state at atmospheric pressure. In other words, the T_{g} corresponds to the temperature where the onset of segmental motion in the chains of the polymer occurs. When an amorphous or semicrystalline polymer is exposed to an increasing temperature, the coefficient of expansion and the heat capacity of the polymer both increase as the temperature is raised, indicating increased molecular motion. As the temperature is raised, the actual molecular volume in the sample remains constant, and so a higher coefficient of expansion points to an increase in free volume associated with the system and therefore increased freedom for the molecules to move. The increasing heat capacity corresponds to an increase in heat dissipation through movement. The T_{g} of a given polymer can be dependent on the heating rate and can be influenced by the thermal history of the polymer. Furthermore, the chemical structure of the polymer heavily influences the glass transition by affecting chain mobility.

An "aliphatic" group is an optionally substituted, straight-chain or branched, saturated or unsaturated hydrocarbon moiety. If unsaturated, the aliphatic group may contain one or more double bonds and/or one or more triple bonds. The aliphatic group is divalent (i.e., -R-) in terms of its attachment to the rest of the compound.

A "heteroaliphatic" group is an aliphatic group that contains at least one heteroatom selected from O, S and N, in the main portion and/or the branch(es) of the hydrocarbon moiety.

A "cycloaliphatic" group is an optionally substituted, saturated or unsaturated, mono- or polycyclic hydrocarbon moiety. If unsaturated, the cycloaliphatic group may contain one or more double bonds in and/or off of one or more rings of the cyclic moiety. The cycloaliphatic group is divalent (i.e., -Cyc-) in terms of its attachment to the rest of the compound, but one or both of the points of attachment may be directly on one or more rings of the cyclic moiety (e.g., -cyclohexyl-) or via one or more groups attached to the ring(s) (e.g., -CH₂-cyclohexyl-CH₂-).

A "heterocycloaliphatic" group is a cycloaliphatic group in which at least one ring in the cyclic moiety contains one or more heteroatoms selected from O, S, and N.

An "aromatic" group is an optionally substituted mono- or polycyclic aromatic moiety. The ring(s) in the moiety may be aromatic or non-aromatic (saturated or unsaturated), but at least one ring in the moiety is aromatic. The aromatic ring(s) in the moiety are carbocyclic, but any non-aromatic ring in the moiety may contain one or more heteroatoms selected from O, S, and N. The aromatic group is divalent (i.e., -Ar-) in terms of its attachment to the rest of the compound, but one or both of the points of attachment may be directly on one or more aromatic or non-aromatic rings of the cyclic moiety (e.g., - phenyl-) or may be via one or more groups attached to the ring(s) (e.g., -CH₂-phenyl-CH₂-).

A "heteroaromatic" group is an aromatic group in which at least one aromatic ring in the aromatic moiety contains one or more heteroatoms selected from O, S, and N.

The aliphatic, heteroaliphatic, cycloaliphatic, heterocycloaliphatic, aromatic and heteroaromatic groups may be substituted or unsubstituted. If substituted, they may contain from I to 5 substituents. The substituents include, but are not limited to: optionally substituted carbon-containing groups, e.g., alkyl, cycloalkyl and aryl; halogen atoms (i.e., F, Cl, Br and I) and optionally substituted halogen-containing groups (e.g., haloalkyl); optionally substituted oxygen-containing groups, e.g., oxo, alcohols and ethers; optionally substituted carbonyl-containing groups, e.g., aldehydes, ketones, carboxy acids, esters, carbonates, thioesters, amides, carbamates and ureas; optionally substituted groups containing carbonyl derivatives, e.g., imines, oximes and thioureas; optionally substituted nitrogen-containing groups, e.g., amines, azides, nitriles and nitro; optionally substituted sulfur-containing groups, e.g., thiols, sulfides, thioethers, sulfoxides, sulfones, sulfonates and sulfonamides; and optionally substituted aromatic or non-aromatic heterocyclic groups containing one or more heteroatoms selected from O, S and N.

### Embodiments of implantable device of the Invention

### Composition and Copolymer

To minimize or prevent adverse side effects associated with a polymer, a drug-delivery stent can be coated with a biodegradable polymer. Examples of such polymers can be derived from highly degradable monomers such as lactide and glycolide. For example, a poly(D,L-lactide-co-glycolide) (PDLGA) copolymer containing 75% D,L-lactide (DLLA) and 25% glycolide (GA) substantially completely degrades within about six months. However, PDLGA copolymers provide unsatisfactory controlled release of hydrophobic drugs such as rapamycin and derivatives thereof (e.g., everolimus). Due to this deficiency, PDLGA copolymers have a narrow process and formulation window with respect to the mass ratio of hydrophobic drug to polymer (D:P). For example, at a D:P mass ratio of 1:1, the PDLGA 75/25 copolymer has a tendency to release everolimus in a burst (> 95% drug release within 24 hours). At D:P ratios of 1:3 and 1:5, PDLGA 75/25 releases everolimus at a substantially lower rate
(< 15% drug release over a period of three days). Only at very specific process and formulation conditions does PDLGA 75/25 provide a controlled release of drugs such as everolimus.

In contrast to PDLGA copolymers, the biodegradable copolymers of the invention provide controlled release of hydrophobic drugs. The inventive copolymers are derived from at least two relatively polar monomers as defined in claim 1 and at least one relatively nonpolar monomer as defined in claim 1. The at least two relatively polar monomers impart a higher Tg and biodegradability to the copolymer. In some embodiments, the relatively polar monomers are relatively hydrophilic. The at least one relatively nonpolar monomer, being relatively hydrophobic, increases the miscibility of a hydrophobic drug with the copolymer. For example, the at least one relatively nonpolar monomer provides a relatively hydrophobic moiety for better phase mix of the copolymer with the hydrophobic drug. The increased miscibility of the hydrophobic drug with the copolymer improves the controlled release of the drug from a polymeric implantable device at lower drug-to-polymer mass ratios (i.e., increasing amounts of polymer relative to drug).

In addition, the at least one relatively nonpolar monomer imparts good mechanical properties (e.g., fracture toughness and flexibility) and good physical properties (e.g., drug permeability) to the copolymer. The mechanical and physical properties of the copolymer can be tuned by appropriate selection of the relatively polar and nonpolar monomers and the ratio and arrangement thereof. For example, the at least one relatively nonpolar monomer and its amount can be selected so as to form a more amorphous copolymer having a lower T_{g}, which would increase the diffusivity of a hydrophobic drug through the copolymer. Further, to improve the biological properties of an implantable device formed of a material comprising the copolymer, one or more biocompatible moieties, non-fouling moieties, and/or biobeneficial materials can be physically or chemically attached to, blended with, or incorporated with the copolymer.

According to the invention the implantable device comprises a coating comprising a composition comprising a biodegradable block copolymer, wherein the copolymer:
is derived from at least two polar monomers selected from glycolide (GA), D-lactide (DLA), L-lactide (LLA), D,L-lactide (DLLA), and meso-lactide (MLA) and at least one nonpolar monomer selected from valerolactone (VL), caprolactone (CL), trimethylene carbonate (TMC), dioxanone (DS), hydroxybutyrate (HB), and hydroxyvalerate (HV);
has a T_{g} from about -150 °C to about 100 °C;
has a polymer number-average molecular weight (Mₙ) from about 10 kDa to about 500 kDa; and
completely degrades, which is the lost of at least about 95 % of its mass, or substantially completely degrades, which is the lost of at least 75% of its mass, within about 12 months;
   and wherein:
each kind of monomer independently has from about 10 to about 5,000 units
   in the copolymer; and
   the molar % of each kind of monomer independently is from about 5% to about 90%.

In a narrower embodiment, optionally in combination with one or more other embodiments described herein, the copolymer:
has a T_{g} from about -100 °C to about 100 °C; and
has an Mₙ from about 20 kDa to about 500 kDa;
   and:
each kind of monomer independently has from about 50 to about 3,000 units in the copolymer;
the molar % of each of the at least two polar monomers independently is from about 5% to about 85%; and
the molar % of each of the at least one nonpolar monomer independently is from about 5% to about 50%.

In one embodiment the copolymer has a T_{g} within a specified range when it is hydrated. In another embodiment, the copolymer has T_{g} within a specified range when it is not hydrated.

The copolymer of the invention has any T_{g} value within the range from -150 °C to 100 °C. The T_{g} of the copolymer can be tuned to a desired value depending on the particular applications of the copolymer. In narrower embodiments, the copolymer can have a T_{g} from about -125 °C to about 100 °C, or from about -100 °C to about 100 °C, or from about -100 °C to about 75 °C, or from about -100 °C to about 50 °C. In certain embodiments, the copolymer can have a T_{g} from about-130 °C to about 90 °C, or from about -110 °C to about 80 °C, or from about -90 °C to about 70 °C, or from about -70 °C to about 60 °C, or from about -50 °C to about 50 °C.

The copolymer can also have a T_{g} in the lower or higher end of the range. For example, in some embodiments, the copolymer can have a T_{g} from about -150 °C to about 0 °C, or from about -130 °C to about -10 °C, or from about -110 °C to about -20 °C, or from about
-90 °C to about -30 °C. In other embodiments, the copolymer can have a T_{g} from about 0 °C to about 100 °C, or from about 10 °C to about 90 °C, or from about 20 °C to about 80 °C, or from about 30 °C to about 70 °C.

A higher T_{g} can increase the strength and rigidity of the copolymer. Strength and rigidity may be important for an implantable device formed of a polymeric material in certain applications, e.g., for a stent so that the stent can support the walls of a vessel.

On the other hand, a lower T_{g} can enhance the fracture toughness and flexibility of the copolymer, increase drug permeability, and improve drug-release control. Toughness and flexibility may be important for a range of aggressive applications of an implantable device, e.g., for a coated stent, such as overlapped stents, stent through stent delivery, and bifurcations. Polymers (e.g., certain glassy, semicrystalline polymers) with too high a T_{g} may be brittle under physiological conditions and fracture during application of the device, exhibiting little or no plastic deformation prior to failure.

The mechanical properties (e.g., strength, rigidity, toughness and flexibility) and physical properties (e.g., T_{g}, crystallinity/amorphousness, degradation rate, drug permeability and drug-release rate) of the copolymer can be tuned by appropriate selection of the polar and nonpolar monomer components; the number, ratio, and arrangement of the monomer components; the length or molecular weight, weight ratio, and arrangement of any segments or blocks of particular monomer component(s) within the copolymer; and any other substances physically or chemically attached to, blended with, or incorporated with the copolymer.

For example, a greater molar % of glycolide (GA), D-lactide (DLA), L-lactide (LLA), D,L-lactide (DLLA) or meso-lactide (MLA) in the copolymer can increase the strength and rigidity of the copolymer. On the other hand, a higher molar % of, e.g., propiolactone, valerolactone (VL), caprolactone (CL), trimethylene carbonate (TMC), dioxanone (DS), or acetals in the copolymer can increase the toughness and flexibility of the copolymer.

As another example, the mechanical and physical properties of the copolymer can be influenced by the selection of the particular relatively polar monomers. For example, copolymers derived from LLA tend be more crystalline than those derived from DLLA. Thus, if, e.g., a greater drug-release rate is desired, then a copolymer can be made more amorphous (i.e., less crystalline) by substituting DLLA for LLA or by incorporating a greater molar % content of DLLA in the copolymer.

Relatively polar and hydrophilic monomers such as GA, DLA, LLA, DLLA and MLA also enhance the degradation rate of the copolymer. Hydrophilic monomers increase the moisture content of the copolymer, which increases its degradation rate. Further, monomers that give acidic degradation products can increase the degradation rate of the copolymer, since the rate of the hydrolysis reaction tends to increase as the pH decreases.

As an example, for faster degradation the copolymer of the invention can contain GA units. When incorporated into a polymer, glycolide hydrolyzes faster than lactide, for the ester bond formed from glycolide is less sterically hindered than that formed from lactide. Further, glycolide units give acidic degradation products that can increase the degradation rate of a GA-containing copolymer.

Accordingly, according to the invention the at least two polar monomers are selected from GA, DLA, LLA, DLLA and MLA, and the at least one nonpolar monomer is selected from VL, CL, TMC, DS, hydroxybutyrate (HB) and hydroxyvalerate (HV). In certain embodiments, the copolymer is a GA-containing terpolymer selected from P(DLA-GA-VL), P(DLA-GA-CL), P(DLA-GA-TMC), P(DLA-GA-DS), P(DLA-GA-HB), P(DLA-GA-HV), P(LLA-GA-VL), P(LLA-GA-CL), P(LLA-GA-TMC), P(LLA-GA-DS), P(LLA-GA-HB), P(LLA-GA-HV), P(DLLA-GA-VL), P(DLLA-GA-CL), P(DLLA-GA-TMC), P(DLLA-GA-DS), P(DLLA-GA-HB), P(DLLA-GA-HV), P(MLA-GA-VL), P(MLA-GA-CL), P(MLA-GA-TMC), P(MLA-GA-DS), P(MLA-GA-HB), and P(MLA-GA-HV). In a more specific embodiment, the copolymer is a GA- and CL-containing terpolymer selected from P(DLA-GA-CL), P(LLA-GA-CL), P(DLLA-GA-CL), and P(MLA-GA-CL).

The body's immune response to a polymeric material forming an implantable device may cause adverse side effects such as late stent thrombosis. To minimize or avoid such adverse immune responses, the copolymer of the invention is designed to completely or substantially completely degrade within 12 months. The copolymer can also be configured to degrade faster in cases where the implantable device is intended to accomplish its functions within a shorter period of time, e.g., locally delivering a drug up to about 6 months or less. Accordingly, in certain embodiments, optionally in combination with one or more other embodiments described herein, the copolymer completely or substantially completely degrades within about 9 months, or within about 6 months, or within about 3 months, or within about 2 months, or within about 1 month (i.e., 30 days).

The physical state of the copolymer can influence its degradation rate and drug permeability. Since a fluid (e.g., water) generally diffuses faster through an amorphous structure than through a crystalline structure, the copolymer can be configured to have a higher degree of amorphousness to increase its degradation rate and drug permeability. Increased water penetration into and water content in an amorphous polymer increases the degradation rate of the polymer and the diffusivity of a drug through the polymer.

Moreover, the physical state of the copolymer can influence its mechanical properties. A more crystalline copolymer can be stronger and more rigid. On the other hand, a more amorphous copolymer can be tougher and more flexible. A more amorphous copolymer can be more "elastomeric" or "rubbery" in that it can exhibit elastic deformation through a greater range of deformation. This characteristic may be desirable when the structure of an implantable device is anticipated to deform during the device's applications, e.g., when a stent that is crimped during delivery expands upon deployment.

To increase its toughness, flexibility, degradation rate and permeability to a hydrophobic drug, the copolymer can be configured to be more amorphous, i.e., be less crystalline. For example, the copolymer of the invention can be designed to have a degree of crystallinity of less than 50%. In some embodiments, the copolymer can have a lower degree of crystallinity depending on the properties desired for the copolymer. In certain embodiments, optionally in combination with one or more other embodiments described herein, the copolymer has a degree of crystallinity of less than 40%, or less than 30%, or less than 20%, or less than 10%, or less than 5%.

In some embodiments, optionally in combination with one or more other embodiments described herein, the biodegradable copolymer of the invention is amorphous. In certain embodiments, the amorphous copolymer has a degree of crystallinity of less than 20%, or less than 15%, or less than 10%, or less than 5%, or less than 3%.

The mechanical properties (e.g., strength, rigidity, toughness and flexibility) and physical properties (e.g., T_{g}, crystallinity/amorphousness, degradation rate, drug permeability, and drug-release rate) of the copolymer can be modified by appropriate selection of the relative amounts of the polar and nonpolar monomers. A higher molar % of the relatively polar and hydrophilic monomers GA, DLA, LLA, DLLA and LLA tends to increase the strength, rigidity. T_{g}, crystallinity and degradation rate of the copolymer (semicrystalline polymers derived from these kinds of monomers can degrade rapidly due to the chemical nature of these monomers). On the other hand, a greater molar % of the relatively nonpolar and hydrophobic monomers VL, CL, TMC, DS, HB and HV tends to lower the T_{g} and degree of crystallinity of the copolymer and increase its toughness, flexibility, degradation rate, miscibility with and permeability to a hydrophobic drug, and improve its controlled release of the hydrophobic drug. For example, a greater molar % of at least one such relatively nonpolar monomer can make the copolymer more amorphous.

In some embodiments, optionally in combination with one or more other embodiments described herein, the at least two relatively polar monomers and the at least one relatively nonpolar monomer each independently have a molar % content in the copolymer from about 5% to about 90%. In other embodiments, the molar % of each of the at least two relatively polar monomers and each of the at least one relatively nonpolar monomer independently is from about 5% to about 85%, or from about 7.5% to about 80%, or from about 10% to about 75%, or from about 12.5% to about 70%, or from about 15% to about 65%. In certain embodiments, the molar % of each of the at least two relatively polar monomers independently is from about 5% to about 90% or from about 5% to about 85%, and the molar % of each of the at least one relatively nonpolar monomer independently is from about 5% to about 70%, or from about 10% to about 70%, or from about 5% to about 50%.

In further embodiments, the biodegradable copolymer is a terpolymer containing 5-50 molar % of GA and 5-70 molar % of a relatively nonpolar monomer such as CL. In narrower embodiments, the copolymer is a terpolymer containing 5-40 molar % of GA and 5-50 molar % of a relatively nonpolar monomer such as CL.

The number of units of particular polar and nonpolar monomers in the copolymer can be adjusted according to various factors such as the desired ratios of the monomers and the desired molecular weight of the copolymer or sections therein. In some embodiments, optionally in combination with one or more other embodiments described herein, each kind of polar and nonpolar monomers independently has from about 10 to about 5,000 units in the copolymer. In narrower embodiments, each kind of polar and nonpolar monomers independently has from about 20 to about 4,500 units, or from about 30 to about 4,000 units, or from about 40 to about 3,500 units, or from about 50 to about 3,000 units in the copolymer.

For forming certain kinds of material (e.g., films, coatings, etc.), the entire copolymer may need to have sufficient molecular weight. Accordingly, the copolymer of the invention has a polymer number-average molecular weight (Mₙ) of at least 10 kDa. In other embodiments, the copolymer has an Mₙ of at least about 20 kDa, or at least about 30 kDa, or at least about 40 kDa, or at least about 50 kDa.

The range of Mₙ of the copolymer can also be influenced by the processing requirements for the particular kind of polymeric material. For example, a polymer with an Mₙ from about 20 kDa to about 500 kDa may be more amenable to being processed into a coating. Thus, in some embodiments, optionally in combination with one or more other embodiments described herein, the inventive copolymer has an Mₙ from about 20 kDa to about 500 kDa, or from about 30 kDa to about 500 kDa, or from about 40 kDa to about 500 kDa, or from about 50 kDa to about 500 kDa. According to the invention, the copolymer has an Mₙ from 10 kDa to 500 kDa.

According to the invention the monomer units are arranged in blocks or segments, where a block or a segment can contain one or more different kinds of monomers. The copolymer can contain a different number of blocks or segments - e.g., one, two, three, four or five blocks or segments. Certain blocks or segments can be the same as or different than other blocks or segments. Further, the monomer units or the blocks or segments can be arranged in an alternating or random manner. For example, a block or a segment can contain two or more different kinds of monomers arranged in an alternating or random manner. As another example, the blocks or segments themselves can be arranged in an alternating or random manner. The monomer units can also be arranged in a graft fashion or in any other manner as is known in the art.

In one embodiment the blocks or segments may or may not be miscible with each other. In one embodiment, the blocks or segments are partially or completely miscible with each other. In another embodiment, the blocks or segments are partially or completely immiscible with one another.

To form discrete phases which are indicative of an immiscible system, the blocks or segments need to be of a certain minimal size. Thus, in some embodiments, the blocks or segments of the copolymer each independently have an Mₙ of at least about 2 kDa, or at least about 3 kDa, or at least about 5 kDa, or at least about 10 kDa.

For a polymer with low permeability of a drug, a high drug/polymer ratio must be employed to get the drug to release. However, a high drug/polymer ratio can lead to a drug-release profile in which most of the drug is released as a burst, and the remaining portion of the drug is released very slowly. On the other hand, a low drug/polymer ratio may result in no or minimal drug release. A higher drug permeability of the polymer can allow better control of drug-release rates at reasonable drug-to-polymer ratios, e.g., where the amount of the polymer is greater than 50% by weight.

The copolymer of the invention is derived from at least one relatively nonpolar monomer (e.g., CL) that increases the miscibility of the copolymer with a hydrophobic drug such as rapamycin or a derivative thereof (e.g., everolimus). The increased miscibility enhances the copolymer's permeability to the hydrophobic drug and improves its controlled release of the drug. Moreover, the at least one relatively nonpolar monomer and its amount in the copolymer can be selected so as to lower the T_{g} of the copolymer and make the copolymer more amorphous, which would also enhance the diffusivity of a hydrophobic drug through the copolymer.

The enhanced permeability of the inventive copolymer to a hydrophobic drug allows for controlled release of the hydrophobic drug at lower drug-to-polymer (D:P) mass ratios. In some embodiments, optionally in combination with one or more other embodiments described herein, the mass ratio of a drug to the copolymer is from about 1:1 to about 1:10. In a narrower embodiment, the D:P mass ratio is from about 1:1 to about 1:5. In more specific embodiments, the D:P mass ratio is about 1:1, or about 1:2, or about 1:3, or about 1:4, or about 1:5. The D:P mass ratios described above apply independently to each of one or more hydrophobic drugs, hydrophilic drugs, biologically active agents, and any other kinds of drugs or agents that the inventive composition can comprise.

### Biocompatible Moieties

To enhance the biocompatibility of the copolymer, some embodiments of the inventive composition, optionally in combination with one or more other embodiments described herein, comprise the copolymer and one or more biologically compatible ("biocompatible") moieties. The biocompatible moieties can be physically or chemically attached to, blended with, or incorporated with the copolymer.

Examples of suitable biocompatible moieties include, but are not limited to, phosphoryl choline; poly(alkylene glycols), e.g., poly(ethylene glycol) (PEG), poly(ethylene oxide), poly(propylene glycol) (PPG), poly(tetramethylene glycol) and poly(ethylene oxide-co-propylene oxide); lactones and lactides, e.g., ε-caprolactone, ß-butyrolactone, δ-valerolactone and glycolide; poly(N-vinyl pyrrolidone); poly(acrylamide methyl propane sulfonic acid) and salts thereof (AMPS and salts thereof); poly(styrene sulfonate); sulfonated dextran; polyphosphazenes; poly(orthoesters); poly(tyrosine carbonate); sialic acid; hyaluronic acid; hyaluronic acid having a stearoyl or palmitoyl substitutent group; copolymers of PEG with hyaluronic acid, hyaluronic acid-stearoyl or hyaluronic acid-palmitoyl; heparin; copolymers of PEG with heparin; a graft copolymer of poly(L-lysine) and PEG; and copolymers thereof. To ensure its renal clearance, the molecular weight of a polymeric biocompatible moiety may be 40 kDa or less, e.g., between about 300 and about 40,000 Daltons, or between about 8,000 and about 30,000 Daltons (e.g., about 15,000 Daltons).

In certain embodiments, optionally in combination with one or more other embodiments described herein, the one or more biocompatible moieties are selected from phosphoryl choline, poly(ethylene oxide), poly(propylene glycol), poly(tetramethylene glycol), poly(ethylene oxide-co-propylene oxide), ε-caprolactone, ß-butyrolactone, δ-valerolactone, glycolide, poly(N-vinyl pyrrolidone), poly(acrylamide methyl propane sulfonic acid) and salts thereof, poly(styrene sulfonate), sulfonated dextran, polyphosphazenes, poly(orthoesters), poly(tyrosine carbonate), sialic acid, hyaluronic acid and derivatives thereof, copolymers of poly(ethylene glycol) (PEG) with hyaluronic acid or derivatives thereof, heparin, copolymers of PEG with heparin, graft copolymers of poly(L-lysine) and PEG, and derivatives and copolymers thereof.

In some embodiments, optionally in combination with one or more other embodiments described herein, the one or more biocompatible moieties specifically cannot be one or more of any of the biocompatible moieties described herein.

### Non-Fouling Moieties

The body's reaction to foreign material could lead to adsorption of proteins on the surface of an implantable device, which could ultimately impair the device's functioning and result in adverse side effects such as thrombosis. A non-fouling moiety provides an implantable device with the ability to resist protein adsorption on its surface. Accordingly, some embodiments of the inventive composition, optionally in combination with one or more other embodiments described herein, comprise the biodegradable copolymer and one or more non-fouling moieties. The one or more non-fouling moieties can be physically or chemically attached to, blended with, or incorporated with the copolymer.

Examples of non-fouling moieties include, without limitation, poly(ethylene glycol) (PEG), poly(propylene glycol), polyethylene oxide, PLURONIC™ surfactants (polypropylene oxide-co-PEG), PEO-PPO surfactants (PLURONIC™ polyols, poly(ethylene oxide-co-propylene oxide)), poly(tetramethylene glycol), amino-terminated PEG, hydroxy functionalized poly(vinyl pyrrolidone), dextran, dextrin, sulfonated dextran, dermatan sulfate, silk-elastin block copolymers, sodium hyaluronate, hyaluronic acid, poly(2-hydroxyethyl methacrylate), dihydroxy poly(styrene sulfonate), poly(3-hydroxypropyl methacrylate,), poly(3-hydroxypropyl methacrylamide), poly(alkoxy methacrylates), poly(alkoxy acrylates), polyarginine peptides (PAP) (e.g., R7), phosphoryl choline, heparin, chondroitan sulfate, glycosaminoglycans, chitosan, and derivatives thereof.

Silk and elastin both are natural proteins. Silk possesses great strength and elastin high flexibility. Their combination in a block copolymer makes the non-fouling moiety very strong and, at the same time, very flexible. Silk-elastin block copolymers can be obtained from Protein Polymer Technologies, Inc. of San Diego, California.

In certain embodiments, optionally in combination with one or more other embodiments described herein, the one or more non-fouling moieties are selected from polyethylene glycol (PEG), polypropylene glycol, Pluronic™ surfactants (polypropylene oxide-co-PEG), poly(2-hydroxyethyl methacrylate) (PHEMA), poly(vinyl alcohol) (PVA), polyalkene oxides, poly(n-propylmethacrylamide), poly(N-vinyl-2-pyrrolidone) (PVP), sulfonated polystyrene, dextran, sulfonated dextran, dextrin, hyaluronic acid, sodium hyaluronate, phosphoryl choline, and derivatives and copolymers thereof.

In some embodiments, optionally in combination with one or more other embodiments described herein, the one or more non-fouling moieties specifically cannot be one or more of any of the non-fouling moieties described herein.

The maximum molecular weight of the at least one non-fouling moiety or, if the non-fouling moiety itself is biodegradable, the maximum molecular weight of the largest fragment formed should be low enough so that it is small enough to pass through the kidneys of an animal (e.g., a human). Thus, in certain embodiments, the molecular weight of the at least one non-fouling moiety or its largest fragment is 40 kDa or less, or 30 kDa or less, or 20 kDa or less.

### Biobeneficial Materials

To improve the biological properties of an implantable device (e.g., enhance its biocompatibility and reduce protein adsorption on its surface), the device can be formed of a material comprising one or more biobeneficial materials. Therefore, some embodiments of the inventive composition, optionally in combination with one or more other embodiments described herein, comprise the biodegradable copolymer and one or more biobeneficial materials. The at least one biobeneficial material may be a polymeric material or a non-polymeric material, and may be biodegradable or non-degradable. In certain embodiments, the at least one biobeneficial material is flexible, biodegradable, biocompatible, non-toxic, non-antigenic and/or non-immunogenic. The at least one biobeneficial material can be physically or chemically attached to, blended with, or incorporated with the copolymer.

The at least one biobeneficial material, if polymeric, may have a relatively low T_{g}, e.g., a T_{g} less than or significantly less than that of the inventive copolymer. In an embodiment, the T_{g} of the biobeneficial material is below body temperature. Having a T_{g} below or significantly below that of the copolymer, the biobeneficial material would be relatively soft as compared to the copolymer. This attribute would, e.g., allow a layer of coating containing the biobenefcial material to fill any surface damages that may arise with an implantable device coated with a layer comprising the copolymer. For example, during radial expansion of a stent, a more rigid copolymer can crack or have surface fractures. A softer biobeneficial material can fill in the crack and fractures.

Examples of biobeneficial materials include, but are not limited to, polyethers (e.g., poly(ethylene glycol) (PEG)); poly(ether esters); co-poly(ether-esters) (e.g. PEO/PLA); polyalkylene oxides (e.g., poly(ethylene oxide) and poly(propylene oxide)); polyalkylene oxalates; polyphosphazenes; phosphoryl choline; choline; poly(aspirin); polymers and copolymers of hydroxyl bearing monomers such as hydroxyethyl methacrylate (HEMA), hydroxypropyl methacrylate (HPMA), hydroxypropylmethacrylamide, poly (ethylene glycol) acrylate (PEGA), PEG methacrylate, 2-methacryloyloxyethylphosphorylcholine (MPC) and n-vinyl pyrrolidone (VP); carboxylic acid bearing monomers such as methacrylic acid (MA), acrylic acid (AA), alkoxymethacrylate, alkoxyacrylate, and 3-trimethylsilylpropyl methacrylate (TMSPMA); copolymers of PEG such as poly(styrene-isoprene-styrene)-PEG (SIS-PEG), polystyrene-PEG, polyisobutylene-PEG, polycaprolactone-PEG (PCL-PEG), PLA-PEG, poly(methyl methacrylate)-PEG (PMMA-PEG), polydimethylsiloxane-co-PEG (PDMS-PEG), and poly(vinylidene fluoride)-PEG (PVDF-PEG); PLURONIC™ surfactants (polypropylene oxide-co-polyethylene glycol); poly(tetramethylene glycol); biomolecules such as fibrin, fibrinogen, cellulose, starch, collagen, dextran, dextrin, hyaluronic acid, fragments and derivatives of hyaluronic acid, heparin, fragments and derivatives of heparin, glycosamino glycan (GAG), GAG derivatives, polysaccharides, elastin, chitosan, and alginate; silicones; and combinations and copolymers thereof.

In certain embodiments, optionally in combination with one or more other embodiments described herein, the one or more biobenefcial materials are selected from fibrin; fibrinogen; cellulose and cellulose derivatives (e.g., cellulose acetate, cellulose butyrate, cellulose acetate butyrate, cellophane, cellulose nitrate, cellulose propionate, cellulose ethers, and carboxymethyl cellulose); starch; pectin; chitosan; elastin; gelatin; alginate and conjugates thereof (e.g., alginate-gelatin, alginate-collagen, alginate-laminin, alginate-elastin, alginate-collagen-laminin and alginate-hyaluronic acid); collagen and conjugates thereof; hyaluronan and derivatives thereof (e.g., methacrylate-modified hyaluronan and NHS ester-modified hyaluronan); hyaluronic acid; sodium hyaluronate; self-assembled peptides (SAPs) (e.g., AcN-RARADADARARADADA-CNH₂ (RAD 16-II), VKVKVKVKV-PP-TKVKVKVKV-NH₂ (MAX-1), and AcN-AEAEAKAKAEAEAKAK-CNH₂ (EAK 16-II)); and derivatives and copolymers thereof.

In another embodiment, the at least one biobeneficial material is a block copolymer having flexible poly(ethylene glycol) and poly(butylene terephthalate) blocks (PEG/PBT) (e.g., PolyActive™). PolyActive™ is intended to include AB, ABA, and BAB copolymers having such segments of PEG and PBT (e.g., poly(ethylene glycol)-block-poly(butylene terephthalate)-block-poly(ethylene glycol) (PEG-PBT-PEG)).

In some embodiments, optionally in combination with one or more other embodiments described herein, the one or more biobeneficial materials specifically cannot be one or more of any of the biobeneficial materials described herein.

### Biologically Active Agents

Other embodiments of the inventive composition, optionally in combination with one or more other embodiments described herein, comprise the biodegradable copolymer and at least one biologically active ("bioactive") agent. The at least one bioactive agent can be physically or chemically attached to, blended with, incorporated with or impregnated in the copolymer, and can include any substance capable of exerting a therapeutic, prophylactic or diagnostic effect for a patient. One of ordinary skill in the art would understand that the terms "bioactive agent" and "drug" can be used interchangeably in appropriate circumstances.

Examples of suitable bioactive agents include, but are not limited to, synthetic inorganic and organic compounds, proteins and peptides, polysaccharides and other sugars, lipids, and DNA and RNA nucleic acid sequences having therapeutic, prophylactic or diagnostic activities. Nucleic acid sequences include genes, antisense molecules that bind to complementary DNA to inhibit transcription, and ribozymes. Some other examples of other bioactive agents include antibodies, receptor ligands, enzymes, adhesion peptides, blood clotting factors, inhibitors or clot dissolving agents such as streptokinase and tissue plasminogen activator, antigens for immunization, hormones and growth factors, oligonucleotides such as antisense oligonucleotides and ribozymes and retroviral vectors for use in gene therapy. The bioactive agents could be designed, e.g., to inhibit the activity of vascular smooth muscle cells. They could be directed at inhibiting abnormal or inappropriate migration and/or proliferation of smooth muscle cells to inhibit restenosis.

In certain embodiments, optionally in combination with one or more other embodiments described herein, the inventive composition comprises at least one biologically active agent selected from antiproliferative, antineoplastic, antimitotic, anti-inflammatory, antiplatelet, anticoagulant, antifibrin, antithrombin, antibiotic, antiallergic and antioxidant substances.

An antiproliferative agent can be a natural proteineous agent such as a cytotoxin or a synthetic molecule. Examples of antiproliferative substances include, but are not limited to, actinomycin D or derivatives and analogs thereof (manufactured by Sigma-Aldrich, or COSMEGEN available from Merck) (synonyms of actinomycin D include dactinomycin, actinomycin IV, actinomycin **I**₁, actinomycin **X**₁, and actinomycin C₁); all taxoids such as taxols, docetaxel, and paclitaxel and derivatives thereof; all olimus drugs such as macrolide antibiotics, rapamycin, everolimus, structural derivatives and functional analogues of rapamycin, structural derivatives and functional analogues of everolimus, FKBP-12 mediated mTOR inhibitors, biolimus, perfenidone, prodrugs thereof, co-drugs thereof, and combinations thereof. Examples of rapamycin derivatives include, but are not limited to, 40-O-(2 -hydroxy)ethyl-rapamycin (generic name everolimus, available from Novartis), 40-O-(2-ethoxy)ethyl-rapamycin (biolimus), 40-O-(3-hydroxy)propyl-rapamycin, 40-O-[2-(2-hydroxy)ethoxy]ethyl-rapamycin, 40-O-tetrazole-rapamycin, 40-epi-(N1-tetrazolyl)-rapamycin (zotarolimus, manufactured by Abbott Labs.), prodrugs thereof, co-drugs thereof, and combinations thereof.

An anti-inflammatory drug can be a steroidal anti-inflammatory drug, a nonsteroidal anti-inflammatory drug (NSAID), or a combination thereof. Examples of anti-inflammatory drugs include, but are not limited to, alclofenac, alclometasone dipropionate, algestone acetonide, alpha amylase, amcinafal, amcinafide, amfenac sodium, amiprilose hydrochloride, anakinra, anirolac, anitrazafen, apazone, balsalazide disodium, bendazac, benoxaprofen, benzydamine hydrochloride, bromelains, broperamole, budesonide, carprofen, cicloprofen, cintazone, cliprofen, clobetasol, clobetasol propionate, clobetasone butyrate, clopirac, cloticasone propionate, cormethasone acetate, cortodoxone, deflazacort, desonide, desoximetasone, dexamethasone, dexamethasone acetate, dexamethasone dipropionate, diclofenac potassium, diclofenac sodium, diflorasone diacetate, diflumidone sodium, diflunisal, difluprednate, diftalone, dimethyl sulfoxide, drocinonide, endrysone, enlimomab, enolicam sodium, epirizole, etodolac, etofenamate, felbinac, fenamole, fenbufen, fenclofenac, fenclorac, fendosal, fenpipalone, fentiazac, flazalone, fluazacort, flufenamic acid, flumizole, flunisolide acetate, flunixin, flunixin meglumine, fluocortin butyl, fluorometholone acetate, fluquazone, flurbiprofen, fluretofen, fluticasone propionate, furaprofen, furobufen, halcinonide, halobetasol propionate, halopredone acetate, ibufenac, ibuprofen, ibuprofen aluminum, ibuprofen piconol, ilonidap, indomethacin, indomethacin sodium, indoprofen, indoxole, intrazole, isoflupredone acetate, isoxepac, isoxicam, ketoprofen, lofemizole hydrochloride, lomoxicam, loteprednol etabonate, meclofenamate sodium, meclofenamic acid, meclorisone dibutyate, mefenamic acid, mesalamine, meseclazone, methylprednisolone suleptanate, momiflumate, nabumetone, naproxen, naproxen sodium, naproxol, nimazone, olsalazine sodium, orgotein, orpanoxin, oxaprozin, oxyphenbutazone, paranyline hydrochloride, pentosan polysulfate sodium, phenbutazone sodium glycerate, pirfenidone, piroxicam, piroxicam cinnamate, piroxicam olamine, pirprofen, prednazate, prifelone, prodolic acid, proquazone, proxazole, proxazole citrate, rimexolone, romazarit, salcolex, salnacedin, salsalate, sanguinarium chloride, seclazone, sermetacin, sudoxicam, sulindac, suprofen, talmetacin, talniflumate, talosalate, tebufelone, tenidap, tenidap sodium, tenoxicam, tesicam, tesimide, tetrydamine, tiopinac, tixocortol pivalate, tolmetin, tolmetin sodium, triclonide, triflumidate, zidometacin, zomepirac sodium, aspirin (acetylsalicylic acid), salicylic acid, corticosteroids, glucocorticoids, tacrolimus, pimecorlimus, prodrugs thereof, co-drugs thereof, and combinations thereof.

Alternatively, the anti-inflammatory agent can be a biological inhibitor of pro-inflammatory signaling molecules. Anti-inflammatory biological agents include antibodies to such biological inflammatory signaling molecules.

In addition, the bioactive agents can be other than antiproliferative or anti-inflammatory agents. The bioactive agents can be any agent that is a therapeutic, prophylactic or diagnostic agent. In some embodiments, such agents can be used in combination with antiproliferative or anti-inflammatory agents. These bioactive agents can also have antiproliferative and/or anti-inflammmatory properties or can have other properties such as antineoplastic, antimitotic, cystostatic, antiplatelet, anticoagulant, antifibrin, antithrombin, antibiotic, antiallergic, and/or antioxidant properties.

Examples of antineoplastics and/or antimitotics include, but are not limited to, paclitaxel (e.g., TAXOL® available from Bristol-Myers Squibb), docetaxel (e.g., Taxotere® from Aventis), methotrexate, azathioprine, vincristine, vinblastine, fluorouracil, doxorubicin hydrochloride (e.g., Adriamycin® from Pfizer), and mitomycin (e.g., Mutamycin® from Bristol-Myers Squibb).

Non-limiting examples of antiplatelet, anticoagulant, antifibrin, and antithrombin agents that can also have cytostatic or antiproliferative properties include sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antagonist antibody, recombinant hirudin, thrombin inhibitors such as ANGIOMAX (from Biogen), calcium channel blockers (e.g., nifedipine), colchicine, fibroblast growth factor (FGF) antagonists, fish oil (e.g., omega 3-fatty acid), histamine antagonists, lovastatin (a cholesterol-lowering drug that inhibits HMG-CoA reductase, brand name Mevacor® from Merck), monoclonal antibodies (e.g., those specific for platelet-derived growth factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), nitric oxide or nitric oxide donors, super oxide dismutases, super oxide dismutase mimetics, 4-amino-2,2,6,6-tetramethylpiperidine-1-oxyl (4-amino-TEMPO), estradiol, anticancer agents, dietary supplements such as various vitamins, and a combination thereof.

Examples of cytostatic substances include, but are not limited to, angiopeptin, angiotensin converting enzyme inhibitors such as captopril (e.g., Capoten® and Capozide® from Bristol-Myers Squibb), cilazapril and lisinopril (e.g., Prinivil® and Prinzide® from Merck).

Non-limiting examples of antiallergic agents include permirolast potassium. Examples of antioxidant substances include, but are not limited to, 4-amino-2,2,6,6-tetramethylpiperidine- -oxyl (4-amino-TEMPO), tris(3,5-di-t-butyl-4-hydroxybenzyl)isocyanurate, 2,2'-methylenebis(4-methyl-6-t-butylphenol), 1,3,5-trimethyl-2,4,6-tris(3,5-di-t-butyl-4-hydroxybenzyl)benzene, butylhydroxytoluene, octadecyl 3,5-di-t-butyl-4-hydroxyhydrocinnamate, 4,4 methylenebis(2,6-di-butylphenol), p,p'-dioctyl diphenylamine, and 1,1,3-tris-(2-methyl-4-hydroxy-5-t-butylphenyl)butane.

Other bioactive agents include anti-infectives such as antiviral agents; analgesics and analgesic combinations; anorexics; antihelmintics; antiarthritics, antiasthmatic agents; anticonvulsants; antidepressants; antidiuretic agents; antidiarrheals; antihistamines; antimigrain preparations; antinauseants; antiparkinsonism drugs; antipruritics; antipsychotics; antipyretics; antispasmodics; anticholinergics; sympathomimetics; xanthine derivatives; cardiovascular preparations including calcium channel blockers and beta-blockers such as pindolol and antiarrhythmics; antihypertensives; diuretics; vasodilators including general coronary vasodilators; peripheral and cerebral vasodilators; central nervous system stimulants; cough and cold preparations, including decongestants; hypnotics; immunosuppressives; muscle relaxants; parasympatholytics; psychostimulants; sedatives; tranquilizers; naturally derived or genetically engineered lipoproteins; and restenoic reducing agents.

Other biologically active agents that can be used include alpha-interferon, genetically engineered epithelial cells, tacrolimus and dexamethasone.

A "prohealing" drug or agent, in the context of a blood-contacting implantable device, refers to a drug or agent that has the property that it promotes or enhances re-endothelialization of arterial lumen to promote healing of the vascular tissue. The portion(s) of an implantable device (e.g., a stent) containing a prohealing drug or agent can attract, bind and eventually become encapsulated by endothelial cells (e.g., endothelial progenitor cells). The attraction, binding, and encapsulation of the cells will reduce or prevent the formation of emboli or thrombi due to the loss of the mechanical properties that could occur if the stent was insufficiently encapsulated. The enhanced re-endothelialization can promote the endothelialization at a rate faster than the loss of mechanical properties of the stent.

The prohealing drug or agent can be dispersed in the body of the bioabsorbable polymer substrate or scaffolding. The prohealing drug or agent can also be dispersed within a bioabsorbable polymer coating over a surface of an implantable device (e.g., a stent).

"Endothelial progenitor cells" refer to primitive cells made in the bone marrow that can enter the bloodstream and go to areas of blood vessel injury to help repair the damage. Endothelial progenitor cells circulate in adult human peripheral blood and are mobilized from bone marrow by cytokines, growth factors, and ischemic conditions. Vascular injury is repaired by both angiogenesis and vasculogenesis mechanisms. Circulating endothelial progenitor cells contribute to repair of injured blood vessels mainly via a vasculogenesis mechanism.

In some embodiments, the prohealing drug or agent can be an endothelial cell (EDC)-binding agent. In certain embodiments, the EDC-binding agent can be a protein, peptide or antibody, which can be, e.g., one of collagen type 1, a 23 peptide fragment known as single chain Fv fragment (scFv A5), a junction membrane protein vascular endothelial (VE)-cadherin, and combinations thereof. Collagen type 1, when bound to osteopontin, has been shown to promote adhesion of endothelial cells and modulate their viability by the down regulation of apoptotic pathways. S.M. Martin, et al., J. Biomed. Mater. Res., 70A: 10-1 9

(2004). Endothelial cells can be selectively targeted (for the targeted delivery of immunoliposomes) using scFv A5. T. Volkel, et al., Biochimica et Biophysica Acta, 1663:158-166 (2004). Junction membrane protein vascular endothelial (VE)-cadherin has been shown to bind to endothelial cells and down regulate apoptosis of the endothelial cells. R. Spagnuolo, et al., Blood, 103:3005-3012 (2004).

In a particular embodiment, the EDC-binding agent can be the active fragment of osteopontin, (Asp-Val-Asp-Val-Pro-Asp-Gly-Asp-Ser-Leu-Ala-Try-Gly). Other EDC-binding agents include, but are not limited to, EPC (epithelial cell) antibodies, RGD peptide sequences, RGD mimetics, and combinations thereof.

In further embodiments, the prohealing drug or agent can be a substance or agent that attracts and binds endothelial progenitor cells. Representative substances or agents that attract and bind endothelial progenitor cells include antibodies such as CD-34, CD-133 and vegf type 2 receptor. An agent that attracts and binds endothelial progenitor cells can include a polymer having nitric oxide donor groups.

The foregoing biologically active agents are listed by way of example and are not meant to be limiting. Other biologically active agents that are currently available or that may be developed in the future are equally applicable.

In some embodiments, optionally in combination with one or more other embodiments described herein, the composition of the invention comprises at least one biologically active agent selected from paclitaxel, docetaxel, estradiol, dexamethasone, clobetasol, nitric oxide donors, super oxide dismutases, super oxide dismutase mimics, 4-amino-2,2,6,6-tetramethylpiperidine-1-oxyl (4-amino-TEMPO), tacrolimus (FK-506), rapamycin (sirolimus), rapamycin derivatives, 40-*O*-(2-hydroxy)ethyl-rapamycin (everolimus), 40-*O*-(2-ethoxy)ethyl-rapamycin (biolimus), 40-*O*-(3-hydroxy)propyl-rapamycin, 40-*O*-[2-(2-hydroxy)ethoxy]ethyl-rapamycin, 40-*O*-tetrazole-rapamycin, 40-epi-(N1-tetrazolyl)-rapamycin (zotarolimus), pimecrolimus, imatinib mesylate, midostaurin, progenitor cell-capturing antibodies, prohealing drugs, prodrugs thereof, co-drugs thereof, and combination thereofs.

In other embodiments, optionally in combination with one or more other embodiments described herein, the inventive composition comprises at least one hydrophobic drug. In certain embodiments, the at least one hydrophobic drug is selected from rapamycin and derivatives thereof such as everolimus, biolimus, 40-*O*-(3-hydroxy)propyl-rapamycin, 40-O-[2-(2-hydroxy)ethoxy]ethyl-rapamycin, 40-*O*-tetrazole-rapamycin, and zotarolimus. In a particular embodiment, the hydrophobic drug is everolimus.

In some embodiments, optionally in combination with one or more other embodiments described herein, the at least one biologically active agent specifically cannot be one or more of any of the bioactive agents or drugs described herein.

### Bioactive Agent-Release Profile

If the implantable device according to the invention comprising the copolymer, contains one or more bioactive agents or drugs, the composition, material or structure can have any one or any combination of a pulse, burst and sustained release profile for each of the bioactive agent(s). For simplicity, the release profile of the bioactive agent(s) will be discussed in the context of an implantable device (e.g., a drug-delivery stent) formed of a material comprising the inventive copolymer.

In some embodiments, optionally in combination with one or more other embodiments described herein, the implantable device provides at least sustained release of each of the one or more bioactive agents. In certain embodiments, the device provides sustained release of each of the bioactive agent(s) over a period up to about 12 months, or up to about 9 months, or up to about 6 months, or up to about 3 months, or up to about 2 months, or up to about 1 month.

As an example, the device can be configured to have a pulse or burst release of a bioactive agent, followed by a sustained release of the same agent. The amount of the bioactive agent released during the pulse or burst phase can be adjusted depending on the needs of the particular therapeutic application. For example, it may be desirable to have a pulse or burst release of some amount of the bioactive agent to initially load up the treatment site with a sufficient amount of the agent, but not such a large amount of the agent that the agent enters systemic circulation and may cause adverse effects. After the initial pulse or burst release, the device can be designed to provide sustained release of the bioactive agent over a period of time depending on the therapeutic needs (e.g., over a period of one month for an anti-proliferative drug or over a period of two months for an anti-inflammatory drug).

The term "pulse release" generally refers to a release profile of a bioactive agent that features a sudden surge in the release rate of the agent. The surge in the release rate of the agent would then disappear within a period of time. A more detailed definition of the term can be found in Encyclopedia of Controlled Drug Delivery, Edith Mathiowitz, Ed., Culinary and Hospitality Industry Publications Services, which is incorporated by reference in its entirety.

In some embodiments, the term "burst release" refers to *in vivo* release of a substantial or large amount of a bioactive agent from an implantable device (e.g., from a coating disposed over the device) in 15 days or less, e.g., within 7 to 14 days. In certain embodiments, a burst release delivers at least about 30%, or at least about 40%, or at least about 50%, or at least about 60%, or at least about 70%, or at least about 80% of a bioactive agent in 15 days or less.

The term "sustained release" generally refers to a release profile of a bioactive agent that can include zero-order release, exponential decay, step-function release or other release profiles that carry over a period of time, e.g., ranging from several days to several weeks, several months or a couple of years. The terms "zero-order release", "exponential decay" and "step-function release" as well as other sustained release profiles are well known in the art. See, e.g., Encyclopedia of Controlled Drug Delivery, Edith Mathiowitz, Ed., Culinary and Hospitality Industry Publications Services.

The release rate of a bioactive agent can be tailored by various means. For example, the release rate of an agent can be tailored by the coating concentration of the agent and the equilibrium water uptake of the barrier if the barrier is formed of a hydrophobic, nonabsorable polymer or the absorption rate if the barrier is formed of an absorbable polymer.

In some embodiments where the implantable device is formed of a material comprising two or more bioactive agents, one of the agents or more than one of them can have any one or a combination of a pulse, burst or sustained release profile. The device (e.g., the coating disposed thereover) can have a release profile that features a pulse or burst release of one or more agents together with a sustained release of the one or more agents. In an embodiment, the device can be configured to have a profile of a pulse or burst release of a first agent and sustained release of the first agent and a second agent. In another embodiment, the device can be designed to have a pulse or burst release of two agents followed by a sustained release of both agents. In yet another embodiment, the device can be configured to provide a pulse release of one or more agents and optionally a sustained release of the same or different agents.

In further embodiments, optionally in combination with one or more other embodiments described herein, the implantable device (e.g., the coating disposed thereover) is capable of simultaneously releasing two or more bioactive agents. Simultaneous delivery means that there is at least some overlap in the release of the agents. Under this embodiment, one of the agents can be released first such as by pulse, burst, or sustained release so long as there is an overlap in release with the second agent.

A coating capable of simultaneously releasing two or more bioactive agents can have a variety of configurations. For example, the coating can have a layer that comprises a mixture of two agents, or it can have two layers, each of which comprises a particular bioactive agent and a polymer that may be the same as or different than the polymer in the other layer.

### Material and Coating

The composition used in the invention comprising the biodegradable copolymer is used to make a material of which a coating for an implantable device is formed. Such a material can comprise any combination of embodiments of the inventive composition or copolymer described herein.

Accordingly, some embodiments used in the invention, optionally in combination with one or more other embodiments described herein, are drawn to a material containing any combination of embodiments of the composition comprising the biodegradable copolymer. For example, the composition forming the material can optionally contain at least one biocompatible moiety, at least one non-fouling moiety, at least one biobeneficial material, at least one biologically active agent, or a combination thereof.

The material of used in the invention can be used to form a portion (e.g., of the body or the surface) of an implantable device (e.g., a stent) or the whole device itself. For example, the material can be used to make a coating that is disposed over at least a portion of the device.

Accordingly, the invention, is directed to an implantable device comprising a coating containing any combination of ernbodiments of the composition comprising the biodegradable copolymer. For example, the composition forming the coating can optionally contain at least one biocompatible moiety, at least one non-fouling moiety, at least one biobeneficial material, at least one biologically active agent, or a combination thereof.

According to the invention the biodegradable copolymer in the coating is derived from at least two polar monomers selected from GA, DLA, LLA, DLLA and MLA, and at least one nonpolar monomer selected from VL, CL, TMC, DS, HB and HV. In certain embodiments, the copolymer is selected from P(DLA-GA-VL), P(DLA-GA-CL), P(DLA-GA-TMC), P(DLA-GA-DS), P(DLA-GA-HB), P(DLA-GA-HV), P(LLA-GA-VL), P(LLA-GA-CL), P(LLA-GA-TMC), P(LLA-GA-DS), P(LLA-GA-HB), P(LLA-GA-HV), P(DLLA-GA-VL), P(DLLA-GA-CL), P(DLLA-GA-TMC), P(DLLA-GA-DS), P(DLLA-GA-HB), P(DLLA-GA-HV), P(MLA-GA-VL), P(MLA-GA-CL), P(MLA-GA-TMC), P(MLA-GA-DS), P(MLA-GA-HB), and P(MLA-GA-HV).

In some embodiments, optionally in combination with one or more other embodiments described herein, the biodegradable copolymer in the coating has a degree of crystallinity of less than 50%, or less than 40%, or less than 30%, or less than 20%, or less than 10%, or less than 5%. In certain embodiments, the copolymer is amorphous. The copolymer has a T_{g} from about -150 °C to about 100 °C, or from about - 100 °C to about 100 °C, or from about -100 °C to about 75 °C, or from about -100 °C to about 50 °C, or from about -50 °C to about 50 °C.

The coating can have a range of thickness and drug-to-polymer ratio depending on, e.g., the desired drug-release rate and degradation rate of the coating. According to the invention , the coating has a thickness of ≤ 6 micron. According to the invention the coating completely or substantially completely degrades within 12 months, or within about 9 months, or within about 6 months, or within about 3 months, or within about 2 months, or within about I month.

If the coating comprises one or more bioactive agents or drugs, the coating can provide any one or any combination of a pulse, burst and sustained release of each of the bioactive agent(s) or drug(s). In some embodiments, optionally in combination with one or more other embodiments described herein, the coating provides sustained release of each of the bioactive agent(s) over a period up to 12 months, or up to 9 months, or up to 6 months, or up to 3 months, or up to 2 months, or up to 1 month.

In some embodiments, optionally in combination with one or more other embodiments described herein, the coating has a mass ratio of each of the drug(s) to the biodegradable copolymer independently ranging from about 1:1 to about 1:10, or from about 1:1 to about 1:5. In more specific embodiments, for each of the drug(s) the coating independently has a drug-to-copolymer mass ratio of about 1:1, or about 1:2, or about 1:3, or about 1:4, or about 1:5.

In further embodiments, optionally in combination with one or more other embodiments described herein, the coating comprises at least one bioactive agent or drug selected from selected from antiproliferative, antineoplastic, antimitotic, anti-inflammatory, antiplatelet, anticoagulant, antifibrin, antithrombin, antibiotic, antiallergic and antioxidant substances. In certain embodiments, the coating comprises at least one bioactive agent or drug selected from paclitaxel, docetaxel, estradiol, dexamethasone, clobetasol, nitric oxide donors, super oxide dismutases, super oxide dismutase mimics, 4-amino-2,2,6,6-tetramethylpiperidine-1-oxyl (4-amino-TEMPO), tacrolimus (FK-506), rapamycin (sirolimus), rapamycin derivatives, 40-O-(2-hydroxy)ethyl-rapamycin (everolimus), 40-O-(2-ethoxy)ethyl-rapamycin (biolimus), 40-O-(3-hydroxy)propyl-rapamycin, 40-O-[2-(2-hydroxy)ethoxy]ethyl-rapamycin, 40-O-tetrazole-rapamycin, 40-epi-(N1-tetrazolyl)-rapamycin (zotarolimus), pimecrolimus, imatinib mesylate, midostaurin, progenitor cell-capturing antibodies, prohealing drugs, prodrugs thereof, co-drugs thereof, and combinations thereof.

### Structure of Coating

According to some embodiments of the invention, optionally in combination with one or more other embodiments described herein, a coating disposed over an implantable device (e.g., a stent) can be a multi-layer structure that can include any of the following four layers or combination thereof:
(1) a primer layer;
(2) a drug-polymer layer (also referred to as a "reservoir" or "reservoir layer") or, alternatively, a polymer-free drug layer;
(3) a topcoat layer; and/or
(4) a finishing coat layer.

Each layer of a stent coating can be disposed over the stent by dissolving the polymer or a blend of polymers in a solvent, or a mixture of solvents, and disposing the resulting polymer solution over the stent by spraying or immersing the stent in the solution. After the solution has been disposed over the stent, the coating is dried by allowing the solvent to evaporate. The process of drying can be accelerated if the drying is conducted at an elevated temperature. The complete stent coating can be optionally annealed at a temperature between about 40 °C and about 150 °C for a period of time between about 5 minutes and about 60 minutes, if desired, to improve the thermodynamic stability of the coating.

To incorporate a bioactive agent (e.g., a drug) into the reservoir layer, the drug can be combined with the polymer solution that is disposed over the stent as described above. Alternatively, if it is desirable to have the stent coating with a fast drug-release rate, a polymer-free reservoir can be made. To fabricate a polymer-free reservoir, the drug can be dissolved in a suitable solvent or mixture of solvents, and the resulting drug solution can be disposed over the stent by spraying or immersing the stent in the drug-containing solution.

Instead of introducing a drug via a solution, the drug can be introduced as a colloid system, such as a suspension in an appropriate solvent phase. To make the suspension, the drug can be dispersed in the solvent phase using conventional techniques used in colloid chemistry. Depending on a variety of factors, e.g., the nature of the drug, those having ordinary skill in the art can select the solvent to form the solvent phase of the suspension, as well as the quantity of the drug to be dispersed in the solvent phase. Optionally, a surfactant can be added to stabilize the suspension. The suspension can be mixed with a polymer solution and the mixture can be disposed over the stent as described above. Alternatively, the drug suspension can be disposed over the stent without being mixed with the polymer solution.

The drug-polymer layer can be applied directly or indirectly over at least a portion of the stent surface to serve as a reservoir for at least one bioactive agent (e.g., drug) that is incorporated into the reservoir layer. The optional primer layer can be applied between the stent and the reservoir to improve the adhesion of the drug-polymer layer to the stent. The optional topcoat layer can be applied over at least a portion of the reservoir layer and serves as a rate-limiting membrane that helps to control the rate of release of the drug. In one embodiment, the topcoat layer can be essentially free from any bioactive agents or drugs. If the topcoat layer is used, the optional finishing coat layer can be applied over at least a portion of the topcoat layer for further control of the drug-release rate and for improving the biocompatibility of the coating. Without the topcoat layer, the finishing coat layer can be deposited directly on the reservoir layer.

The copolymer used in the invention derived from at least two polar monomers and at least one nonpolar monomer can be used to form any layer of the coating. In one embodiment, the copolymer forms the drug reservoir, or drug matrix, layer of the coating. In another embodiment, the copolymer forms the topcoat layer. In yet another embodiment, the copolymer forms the finishing coat layer. In still another embodiment, the copolymer forms the primer layer. In a further embodiment, the copolymer forms any combination of the primer, drug reservoir, topcoat and finishing coat layers of the coating.

The process of the release of a drug from a coating having both topcoat and finishing coat layers includes at least three steps. First, the drug is absorbed by the polymer of the topcoat layer at the drug-polymer layer/topcoat layer interface. Next, the drug diffuses through the topcoat layer using the void volume between the macromolecules of the topcoat layer polymer as pathways for migration. The drug then arrives at the topcoat layer/finishing layer interface. Finally, the drug diffuses through the finishing coat layer in a similar fashion, arrives at the outer surface of the finishing coat layer, and desorbs from the outer surface. At this point, the drug is released into the blood vessel or surrounding tissue. Consequently, a combination of the topcoat and finishing coat layers, if used, can serve as a rate-limiting barrier. The drug can be released by virtue of the degradation, dissolution, and/or erosion of the layer(s) forming the coating, or via migration of the drug through degradable and/or non-degradable polymeric layer(s) into a blood vessel or tissue.

In one embodiment, any or all of the layers of the stent coating can be made of biodegradable polymer(s), biostable polymer(s), or a combination thereof. In another embodiment, the outermost layer of the coating can be limited to biodegradable polymer(s), biostable polymer(s), or a combination thereof.

To illustrate in more detail, in a stent coating having all four layers described above (i.e., the primer, the reservoir layer, the topcoat layer and the finishing coat layer), the outermost layer is the finishing coat layer, which can be made of biodegradable polymer(s), biostable polymer(s), or a combination thereof. The remaining layers (i.e., the primer, the reservoir layer and the topcoat layer) optionally can also be fabricated of biodegradable polymer(s), biostable polymer(s), or a combination thereof. The polymer(s) in a particular layer may be the same as or different than those in any of the other layers.

If a finishing coat layer is not used, the topcoat layer can be the outermost layer and can be made of biodegradable polymer(s), biostable polymer(s), or a combination thereof. In this case, the remaining layers (i.e., the primer and the reservoir layer) optionally can also be fabricated of biodegradable polymer(s), biostable polymer(s), or a combination thereof. The polymer(s) in a particular layer may be the same as or different than those in any of the other layers.

If neither a finishing coat layer nor a topcoat layer is used, the stent coating could have only two layers - the primer and the reservoir. In such a case, the reservoir is the outermost layer of the stent coating and can be made of biodegradable polymer(s), biostable polymer(s), or a combination thereof. The primer optionally can also be fabricated of biodegradable polymer(s), bistable polymer(s), or a combination thereof. The two layers may be made from the same or different polymers.

Increased rate of degradation, erosion, absorption and/or resorption of biologically degradable, erodable, absorbable and /or resorbable polymer(s) can lead to an increased rate of release of a drug due to the gradual disappearance of the polymer(s) that form the reservoir, the topcoat layer, and/or the finishing coat layer. Through appropriate selection of biodegradable polymer(s), biostable polymer(s) or a combination thereof, a stent coating can be engineered to provide either fast or slow release of a drug, as desired. Those having ordinary skill in the art can determine whether a stent coating having slow or fast drug-release rate is advisable for a particular drug. For example, fast release may be recommended for stent coatings loaded with antimigratory drugs, which often need to be released within 1 to 2 weeks. For anti-proliferative and anti-inflammatory drugs, slower release may be desired, e.g., up to 30-day and 60-day release times, respectively.

Any layer of a stent coating can contain any amount of a bioabsorbable polymer and/or a biocompatible polymer, or a blend of more than one such polymer. Non-limiting examples of bioabsorbable polymers and biocompatible polymers include polyacrylates, e.g., poly(butyl methacrylate), poly(ethyl methacrylate), poly(ethyl methacrylate-co-butyl methacrylate), poly(acrylonitrile), poly(ethylene-co-methyl methacrylate), poly(acrylonitrile-co-styrene) and poly(cyanoacrylates); fluorinated polymers and/or copolymers, e.g., poly(vinylidene fluoride) and poly(vinylidene fluoride-co-hexafluoro propylene); poly(N-vinyl pyrrolidone); polydioxanone; polyorthoesters; polyanhydrides; poly(glycolic acid); poly(glycolic acid-co-trimethylene carbonate); polyphosphoesters; polyphosphoester urethanes; poly(amino acids); poly(trimethylene carbonate); poly(iminocarbonates); co-poly(ether-esters); polyalkylene oxalates; polyphosphazenes; biomolecules, e.g., fibrin, fibrinogen, cellulose, cellophane, starch, collagen, hyaluronic acid, and derivatives thereof (e.g., cellulose acetate, cellulose butyrate, cellulose acetate butyrate, cellulose nitrate, cellulose propionate, cellulose ethers, and carboxymethyl cellulose); polyurethanes; silicones; polyesters; polyolefins;polyisobutylene and ethylene-alphaolefin copolymers; vinyl halide polymers and copolymers, e.g., polyvinyl chloride; polyvinyl ethers, e.g., polyvinyl methyl ether; polyvinylidene chloride; polyvinyl ketones; polyvinyl aromatics, e.g., polystyrene; polyvinyl esters, e.g., polyvinyl acetate; copolymers of vinyl monomers with each other and olefins, e.g., poly(ethylene-co-vinyl alcohol) (EVAL); ABS resins; poly(ethylene-co-vinyl acetate); polyamides, e.g., Nylon 66 and polycaprolactam; alkyd resins; polycarbonates; polyoxymethylenes; polyimides; polyethers, epoxy resins; polyurethanes; rayon; rayon-triacetate; and copolymers thereof.

Any layer of a stent coating can also contain any amount of a non-degradable polymer, or a blend of more than one such polymer. Non-limiting examples of non-degradable polymers include methylmethacrylate, ethylmethacrylate, butylmethacrylate, 2-ethylhexylmethacrylate, laurylmethacrylate, hydroxyl ethyl methacrylate, polyethylene glycol (PEG) acrylate, PEG methacrylate, 2-methacryloyloxyethylphosphorylcholine (MPC) and N-vinyl pyrrolidone, methacrylic acid, acrylic acid, hydroxypropyl methacrylate, hydroxypropylmethacrylamide, 3-trimethylsilylpropyl methacrylate, and copolymers thereof.

### Implantable Device

A portion of the implantable device or the whole device itself can be formed of the material containing any combination of embodiments of the composition comprising the biodegradable copolymer as defined in claim 1. For example, a portion of the body or the whole body of the device can be formed of the inventive material. As another example, a coating containing any combination of embodiments of the composition comprising the biodegradable copolymer can be disposed over at least a portion of the implantable device.

The coating is disposed over at least a portion of the device. For example, the implantable device can be formed of a coating comprising a composition that can optionally contain at least one biocompatible moiety, at least one non-fouling moiety, at least one biobeneficial material, at least one biologically active agent, or a combination thereof

The implantable devie comprises coating comprising a biodegradable copolymer that is derived from at least two polar monomers selected from GA, DLA, LLA, DLLA and MLA, and at least one nonpolar monomer selected from VL, CL, TMC, DS, HB and HV. The device is coated with a coating that has a thickness of ≤ 6 micron.

The implantable device comprises a coating that completely or substantially completely degrades within 12 months, or within about 9 months, or within about 6 months, or within about 3 months, or within about 2 months, or within about 1 month. The inventive coating disposed over the device can provide sustained release of one or more bioactive agents over a period up to 12 months, or up to 9 months, or up to 6 months, or up to 3 months, or up to 2 months, or up to month.

In some embodiments, optionally in combination with one or more other embodiments described herein, a portion of the device (e.g., a coating disposed over the device) or the whole device itself can be formed of such polymers and any other substances described herein.

Non-limiting examples of implantable devices include stents (e.g., coronary stents and peripheral stents), grafts (e.g., aortic grafts, arterio-venous grafts, vascular grafts and by-pass grafts), stent-grafts, catheters, guidewires, leads and electrodes for pacemakers and defibrillators, endocardial leads (e.g., FINELINE and ENDOTAK, available from Abbott Vascular, Santa Clara, California), clips (e.g., anastomotic clips), shunts (e.g., cerebrospinal fluid and axius coronary shunts), closure devices (e.g., arterial and patent foramen ovale closure devices), valves (e.g., artificial heart valves), ventricular assist devices, artificial heart, and blood oxygenators. Furthermore, the material used in the invention containing any combination of embodiments of the composition comprising the biodegradable copolymer as defined in claim 1 can be use to make other types of substrates including, e.g., nanofibers, sustained-release small molecule or protein formulations, microspheres, and particles (e.g., drug-delivery particles, microparticles and nanoparticles).

In certain embodiments, optionally in combination with one or more other embodiments described herein, the implantable device is selected from stents, grafts, stent-grafts, catheters, leads, electrodes, clips, shunts, closure devices, valves and particles. In a specific embodiment, the implantable device is a stent. The stent may be balloon-expandable or self-expandable. Moreover, the stent can be intended for any vessel in the body, e.g., neurological, carotid, vein graft, synthetic graft, arteriovenous anastamosis, coronary, aortic renal, iliac, femoral, popliteal vasculature and urethral passages.

The implantable device and its underlying structure can be of virtually any design. As an example, the device can be of any size or shape that is suitable for the device's intended functions. As another example, a portion of the device, or the whole device itself, can be made of a metallic material, an alloy, a polymeric material, any other type of material, or a combination thereof, as is known in the art. For instance, a polymeric material comprising any combination of embodiments of the composition as defined in claim 1 can be used to make a portion of the implantable device or the whole device itself. For example, the body of a stent can be made of a metal or an alloy, and the inventive composition can be coated over the stent.

Non-limiting examples of metallic materials and alloys suitable for fabricating implantable devices include cobalt-chromium alloys (e.g., ELGILOY), "L-605", stainless steel (316L), "MP35N," "MP20N," ELASTINITE (Nitinol), tantalum, tantalum-based alloys, nickel-titanium alloys, platinum, platinum-based alloys (e.g., platinum-iridium alloy), iridium, gold, magnesium, titanium, titanium-based alloys, zirconium-based alloys, or combinations thereof. "L-605" is a trade name for an alloy of cobalt, chromium, tungsten, nickel and iron available as Haynes 25 from Haynes International (Kokomo, Indiana). "L-605" consists of 51 % cobalt, 20% chromium, 15% tungsten, 10% nickel and 3% iron. "MP35N" and "MP20N" are trade names for alloys of cobalt, nickel, chromium and molybdenum available from Standard Press Steel Co. (Jenkintown, Pennsylvania). "MP35N" consists of 35% cobalt, 35% nickel, 20% chromium and 10% molybdenum. "MP20N" consists of 50% cobalt, 20% nickel, 20% chromium and 10% molybdenum.

If a polymeric material is used to make a coating of the implantable device or the whole device itself, the polymeric material can comprise any combination of embodiments of the inventive composition as defined in claim 1, e.g., the biodegradable copolymer of the invention, a blend of different types of polymers, a blend of polymer(s) and additional substance(s), or a combination thereof. Further, additional polymer(s) and/or additional substance(s) can be physically or chemically attached to the underlying copolymer forming the device or a portion thereof. The additional polymer(s) and/or additional substance(s) that can be physically or chemically attached to, blended with, or incorporated with the underlying copolymer include, but are not limited to, biocompatible polymers, bioabsorbable polymers, biocompatible moieties, non-fouling moieties, biobeneficial substances and materials, and bioactive agents. To enhance the mechanical characteristics (e.g., strength and rigidity) of an implantable device made substantially of a polymeric material, the device can be supported by additional structure(s) (e.g., struts in the case of stents made substantially of a polymeric material).

Non-limiting examples of polymers that can be used to fabricate an implantable device include poly(N-acetylglucosamine) (Chitin), Chitosan, poly(hydroxyvalerate), poly(lactide-co-glycolide), poly(hydroxybutyrate), poly(hydroxybutyrate-co-valerate), polyorthoester, polyanhydride, poly(glycolic acid), poly(glycolide), poly(L-lactic acid), poly(L-lactide), poly(D,L-lactic acid), poly(L-lactide-co-glycocide), poly(D,L-lactide), poly(caprolactone), poly(trimethylene carbonate), polyethylene amide, polyethylene acrylate, poly(glycolic acid-co(trimethylence carbonate), co-poly(ether-esters) (e.g., PEO/PLA), polyphosphazenes, biomolecules (e.g., fibrin, fibrinogen, cellulose, starch, collagen and hyaluronic acid), polyurethanes, silicones, polyesters, polyolefins, polyisobutylene and ethylene-alphaolefin copolymers, acrylic polymers and copolymers other than polyacrylates, vinyl halide polymers and copolymers (e.g., polyvinyl chloride), polyvinyl ethers (e.g., polyvinyl methyl ether), polyvinylidene halides (e.g., polyvinylidene chloride), polyacrylonitrile, polyvinyl ketones, polyvinyl aromatics (e.g., polystyrene), polyvinyl esters (e.g., polyvinyl acetate), acrylonitrile-styrene copolymers, ABS resins, polyamides (e.g., Nylon 66 and polycaprolactam), polycarbonates, polyoxymethylenes, polyimides, polyethers, polyurethanes, rayon, rayon-triacetate, cellulose and derivates thereof (e.g., cellulose acetate, cellulose butyrate, cellulose acetate butyrate, cellophan,e, cellulose nitrate, cellulose propionate, cellulose ethers, and carboxymethyl cellulose), and copolymers thereof.

Additional representative examples of polymers that may be suited for fabricating an implantable device include ethylene vinyl alcohol copolymer (commonly known by the generic name EVOH or by the trade name EVAL), poly(butyl methacrylate), poly(vinylidene fluoride-co-hexafluoropropylene) (e.g., SOLEF 21508, available from Solvay Solexis PVDF ATOFINA Chemicals of Philadelphia, Pennsylvania), poly(tetrafluoroethylene-co-hexafluoropropylene-co-vinylidene fluoride), ethylene-vinyl acetate copolymers, and polyethylene glycol.

### Method of Fabricating Implantable Device

The method comprises depositing over at least a portion of the implantable device a coating containing any combination of embodiments of the composition comprising the biodegradable copolymer, as defined in claim 1.

Accordingly, the method comprises disposing over at least a portion of an implantable device a coating containing any combination of embodiments of the composition comprising the biodegradable copolymer as defined in claim 1. For example, the method comprises depositing over at least a portion of an implantable device a coating comprising a composition that can optionally contain at least one biocompatible moiety, at least one non-fouling moiety, at least one biobeneficial material, at least one biologically active agent, or a combination thereof.

The method can deposit a coating having a range of thickness over an implantable device. The method deposits over at least a portion of the implantable device coating that has a thickness of 6 micron.

In some embodiments, the method is used to fabricate an implantable device comprising a coating, said implantable device selected from stents, grafts, stent-grafts, catheters, leads, electrodes, clips, shunts, closure devices, valves, and particles. In a particular embodiment, the method is used to fabricate a stent comprising a coating.

### Method of Treating or Preventing Disorders

An implantable device formed of a material or coating containing any combination of embodiments of the composition comprising the biodegradable copolymer as defined in claim 1 can be used to treat, prevent or diagnose various conditions or disorders. Examples of such conditions or disorders include, but are not limited to, vascular and related conditions or disorders such as atherosclerosis, thrombosis, restenosis, hemorrhage, vascular dissection, vascular perforation, vascular aneurysm, vulnerable plaque, chronic total occlusion, patent foramen ovale, claudication, anastomotic proliferation of vein and artificial grafts, arteriovenous anastamoses, bile duct obstruction, ureter obstruction and tumor obstruction.

Accordingly, some embodiments, optionally in combination with one or more other embodiments described herein, are drawn to a method of treating, preventing or diagnosing a condition or disorder in a patient, comprising implanting in the patient an implantable device formed of a material or coating containing any combination of embodiments of the composition comprising the biodegradable copolymer as defined in claim 1. The implantable device is formed of a coating comprising a composition that can optionally contain at least one biocompatible moiety, at least one non-fouling moiety, at least one biobeneficial material, at least one biologically active agent, or a combination thereof.

In certain embodiments, optionally in combination with one or more other embodiments described herein, the method treats, prevents or diagnoses a condition or disorder selected from atherosclerosis, thrombosis, restenosis, hemorrhage, vascular dissection, vascular perforation, vascular aneurysm, vulnerable plaque, chronic total occlusion, patent foramen ovale, claudication, anastomotic proliferation of vein and artificial grafts, arteriovenous anastamoses, bile duct obstruction, ureter obstruction, tumor obstruction, and combinations thereof. In a more specific embodiment, the condition or disorder is atherosclerosis, thrombosis, restenosis, vulnerable plaque, or a combination thereof.

A portion of the implantable device or the whole device employed in the method can be formed of a material containing any combination of embodiments of the composition comprising the biodegradable copolymer, as described herein. The material is a coating disposed over at least a portion of the device, wherein the coating contains any combination of embodiments of the composition comprising the biodegradable copolymer as defined in claim 1. For example, the coating can optionally contain at least one biocompatible moiety, at least one non-fouling moiety, at least one biobeneficial material, at least one biologically active agent, or a combination thereof.

The biodegradable copolymer in the coating is derived from at least two polar monomers selected from GA, DLA, LLA, DLLA and MLA, and at least one nonpolar monomer selected from VL, CL, TMC, DS, HB and HV. In certain embodiments, the copolymer is a GA-containing terpolymer selected from P(DLA-GA-VL), P(DLA-GA-CL), P(DLA-GA-TMC), P(DLA-GA-DS), P(DLA-GA-HB), P(DLA-GA-HV), P(LLA-GA-VL), P(LLA-GA-CL), P(LLA-GA-TMC), P(LLA-GA-DS), P(LLA-GA-HB), P(LLA-GA-HV), P(DLLA-GA-VL), P(DLLA-GA-CL), P(DLLA-GA-TMC), P(DLLA-GA-DS), P(DLLA-GA-HB), P(DLLA-GA-HV), P(MLA-GA-VL), P(MLA-GA-CL), P(MLA-GA-TMC), P(MLA-GA-DS), P(MLA-GA-HB), and P(MLA-GA-HV). In a more specific embodiment, the biodegradable copolymer in the coating is a GA- and CL-containing terpolymer selected from P(DLA-GA-CL), P(LLA-GA-CL), P(DLLA-GA-CL), and P(MLA-GA-CL).

The biodegradable copolymer derived from at least two polar monomers and at least one nonpolar monomer can be used to form any layer of the coating. In one embodiment, the copolymer forms the drug reservoir, or drug matrix, layer of the coating. In another embodiment, the copolymer forms the topcoat layer. In yet another embodiment, the copolymer forms the finishing coat layer. In still another embodiment, the copolymer forms the primer layer. In a further embodiment, the copolymer forms any combination of the primer, drug reservoir, topcoat and finishing coat layers of the coating.

In further embodiments of the method, the biodegradable copolymer in the coating disposed over the implantable device has a degree of crystallinity of less than 50%, or less than 40%, or less than 30%, or less than 20%, or less than 10%, or less than 5%. In certain embodiments, the copolymer is amorphous. The copolymer has a T_{g} from 150 °C to 100 °C, or from about -100 °C to about 100 °C, or from about - 100 °C to about 75 °C, or from about -100 °C to about 50°C, or from about -50 °C to about 50 °C.

The coating over the device that is used in the method can have a range of thickness and drug-to-polymer ratio depending on, e.g., the desired drug-release rate and degradation rate of the coating. The coating has a thickness of 6 micron. In other embodiments, the coating completely or substantially completely degrades within about 12 months, or within about 9 months, or within about 6 months, or within about 3 months, or within about 2 months, or within about 1 month.

The implantable device coated with the composition of the invention can provide any one or any combination of a pulse, burst and sustained release of one or more bioactive agents or drugs. In some embodiments, the coated device provides at least sustained release of each of the bioactive agent(s). In certain embodiments, the coated device provides sustained release of each of the bioactive agent(s) over a period up to 12 months, or up to 9 months, or up to 6 months, or up to 3 months, or up to 2 months, or up to 1 month.

In some embodiments, the coating over the implantable device has a mass ratio of each of the bioactive agent(s) to the biodegradable copolymer independently ranging from about 1:1 to about 1:10, or from about 1:1 to about 1:5. In more specific embodiments, for each of the bioactive agent(s) the coating independently has a bioactive agent-to-copolymer mass ratio of about 1:1, or about 1:2, or about 1:3, or about 1:4, or about 1:5.

In some embodiments of the method, the coating over the device comprises one or more bioactive agents selected from antiproliferative, antineoplastic, antimitotic, anti-inflammatory, antiplatelet, anticoagulant, antifibrin, antithrombin, antibiotic, antiallergic and antioxidant substances. In certain embodiments, the coating comprises at least one bioactive agent selected from paclitaxel, docetaxel, estradiol, dexamethasone, clobetacol, nitric oxide donors, super oxide dismutases, super oxide dismutase mimics, 4-amino-2,2,6,6-tetramethylpiperidine-1-oxyl (4-amino-TEMPO), tacrolimus (FK-506), rapamycin (sirolimus), rapamycin derivatives, 40-*O*-(2-hydroxy)ethyl-rapamycin (everolimus), 40-*O*-(2-ethoxy)ethyl-rapamycin (biolimus), 40-*O*-(3-hydroxy)propyl-rapamycin, 40-*O*-[2-(2-hydroxy)ethoxy]ethyl-rapamycin, 40-*O*-tetrazole-rapamycin, 40-epi-(N1-tetrazolyl)-rapamycin (zotarolimus), pimecrolimus, imatinib mesylate, midostaurin, progenitor cell-capturing antibodies, prohealing drugs, prodrugs thereof, co-drugs thereof, and combinations thereof.

In other embodiments, the coating over the device comprises one or more hydrophobic drugs. In certain embodiments, the coating comprises at least one hydrophobic drug selected from rapamycin and derivatives thereof such as everolimus, biolimus, 40-*O*-(3-hydroxy)propyl-rapamycin, 40-*O*-[2-(2-hydroxy)ethoxy]ethyl-rapamycin, 40-*O*-tetrazole-rapamycin, and zotarolimus. In a particular embodiment, the at least one hydrophobic drug is everolimus.

In certain embodiments, the implantable device employed in the method is selected from stents, grafts, stent-grafts, catheters, leads, electrodes, clips, shunts, closure devices, valves, and particles. In a specific embodiment, the implantable device is a stent.

### Examples

The examples set forth below are shown for the sole purpose of further illustrating embodiments of the present invention and are in no way meant to limit the invention. The following prophetic examples are given to aid in understanding the invention, but it is to be understood that the invention is not limited to the particular materials or procedures of the examples.

### Synthesis of Biodegradable Copolymers

The biodegradable copolymer used in the invention can be prepared by any method of polymerization known in the art. Methods of polymerization include, but are not limited to, solution-based polymerization and melt-phase polymerization. In solution-based polymerization, all the reactive components involved in the polymerization reaction are partially or completely dissolved in a solvent.

The copolymer used in the invention can be synthesized by standard methods known to those having ordinary skill in the art, e.g., by ring-opening polymerization (ROP) with the corresponding monomers of the copolymer and using an initiator. ROP can be catalyzed by an organic or inorganic acid (e.g., a Lewis acid), an organic (e.g., a tertiary amine base) or inorganic base (e.g., a Lewis base), an organometallic reagent, and/or heat, if necessary and if compatible with the reactants and product(s) of the reaction. For example, zirconium catalysts such as zirconium acetylacetone, zinc catalysts such as diethylzinc and zinc L-lactate, and tin catalysts such as stannous octoate and tin triflates are particularly suitable for the synthesis of biodegradable polyesters.

In some embodiments, the initiator employed in the synthesis of the copolymer has at least one active end group that is a hydroxyl, amino or thiol group. If the initiator has only one active end group, then the polymer grows only at one end. On the other hand, if the initiator has two active end groups, then a polymerization reaction occurs at both ends of the polymer. In an embodiment, the initiator is a diol, in which one of the hydroxyl end groups may optionally be protected. In another embodiment, the initiator is a diamine, in which one of the amino end groups may optionally be protected. In yet another embodiment, the initiator is a dithiol, in which one of the thiol end groups may optionally be protected. In further embodiments, the dihydroxy, dianiino or dithiol initiator is C₂-C₂₄ and contains an optionally substituted aliphatic, heteroaliphatic, cycloaliphatic, heterocycloaliphatic, aromatic or heteroaromatic group, or a combination thereof. In other embodiments, the initiator is a diol selected from 1,2-ethanediol, 1,3-propanediol, 1,4-butanediol, 1,5-pentanediol, 1,6-hexanediol, 1,7-heptanediol, 1,8-octanediol, diethylene glycol, triethylene glycol, tetraethylene glycol, poly(ethylene glycol), poly(propylene glycol), poly(tetramethylene glycol), and poly(caprolactone) did.

Accordingly, it is disclosed a method of preparing the composition, comprising performing ring-opening polymerization (ROP) reactions with an initiator, at least two polar monomers and at least one nonpolar monomer, wherein:
the initiator has one or two active hydroxyl, amino or thiol end groups;
the initiator can do the initial ROP reaction with a polar monomer or a nonpolar monomer;
the ROP reaction with each different kind of monomer can occur in any order and for any number of times; and
a particular ROP reaction can occur in the presence of only one kind of monomer or in the presence of two or more different kinds of monomers.

Various embodiments of the composition comprising the biodegradable copolymer can be prepared by optionally:
blending or physically or chemically attaching at least one biocompatible moiety with or to the copolymer;
blending or physically or chemically attaching at least one non-fouling moiety with or to the copolymer;
blending or physically or chemically attaching at least one biobeneficial material with or to the copolymer; and/or
blending, physically or chemically attaching, or impregnating at least one biologically active agent with, to, or in the copolymer.

One example of the synthesis of a biodegradable copolymer is the synthesis of a P(CL-GA-DLLA) terpolymer via ROP in **Scheme 1**. In this example, mono-protected 1,6-hexanediol is the initiator. It initiates ROP with ε-caprolactone (CL) to form PCL. The hydroxyl end group of PCL then initiates ROP with glycolide (GA) to generate P(CL-GA). Similarly, the hydroxyl end group of P(CL-GA) in turn initiates ROP with D,L-lactide (DLLA) to furnish P(CL-GA-DLLA).

In the example illustrated in Scheme 1, it is understood that a particular ROP reaction with a particular kind of monomer can occur any number of times. Therefore, the variables m, n and p can be any integer ≥ 1. For example, each of these variables can independently be from 10 to 5,000, or from 20 to 4,500, or from 30 to 4,000, or from 40 to 3,500, or from 50 to 3,000. The order of ROP reactions depicted in Scheme 1 is merely illustrative. The ROP reactions involving the three different kinds of monomer can occur in any order. Moreover, a particular ROP reaction can be random by being performed in the presence of two or more different kinds of monomers.

If a particular ROP reaction with a particular kind of monomer occurs multiple times, a block or segment derived from that monomer can be created. The block or segment can be of a certain length or molecular weight, and can be arranged in a random or alternating fashion with another block or segment derived from another kind of monomer. Further, a block or segment can be derived from two or more different kinds of monomers, wherein each kind of monomer undergoes a particular ROP reaction a certain number of times. These ROP reactions can also alternate in forming a block or segment. In addition, a random block or segment can be created by conducting an ROP reaction in the presence of two or more different kinds of polymers any number of times, optionally conducting another ROP reaction in the presence of two or more other kinds of polymers any number of times, and so on.

If desired, the protected "left" end of the copolymer in Scheme 1 can remain protected after the polymerization reactions have been completed, and the hydroxyl group at the "right" end can be functionalized in any desired manner. Alternatively, deprotection of the left end of the copolymer allows this end to be functionalized in any desired fashion, with appropriate pre-protection of the right end group, if desired. The use of protecting (or blocking) groups in organic synthesis is well known in the art.

For example, both hydroxyl end groups can be conjugated to a dihydroxyaryl group to enhance the adhesion of the copolymer to a metal surface. The dihydroxyaryl group can contain, e.g., an *ortho*-dihydroxyphenyl moiety such as 1,2-dihydroxyphenyl and 3,4-dihydroxyphenyl. 3,4-Dihydroxyphenyl-containing compounds include, e.g., dopamine and 3,4-dihydroxyhydrocinnamic acid. Dopamine could be conjugated to the hydroxyl end groups of the copolymer, e.g., via coupling with 1,1'-carbonyldiimidazole. 3,4-Dihydroxy-hydrocinnamic acid could be conjugated to the hydroxyl end groups, e.g., by conversion of the cinnamic acid to the N-succidimyl ester or by use of dicyclohexylcarbodiimide (DCC) and
4-(dimethylamino)pyridinium (DPTS). Alternatively, conjugation of the cinnamic acid could be effected via a Mitsunobu reaction using triphenylphosphine and diethyl azodicarboxylate (DEAD) or diisopropyl azodicarboxylate (DIAD). Conjugation of a dihydroxyaryl group to an active end group (e.g., a hydroxyl, amino or thiol group) could also be effected using other reagents and methods, as is known in the art.

As another example, either the left or the right hydroxyl end group, or both end groups, independently can be attached to a biocompatible moiety, a non-fouling moiety, a biobeneficial material, and/or a bioactive agent. Monomers different than those shown in Scheme 1 and bearing a protected side group can also be used in synthesizing the copolymer. After completion of the polymerization reactions, the side groups can be deprotected and functionalized as desired, e.g., by attaching them to a biocompatible moiety, a non-fouling moiety, a biobeneficial material, and/or a bioactive agent.

As a further example, either the left or the right hydroxyl end group, or both end groups, can undergo ROP with CL, GA, DLLA or a different polar or nonpolar monomer to further develop the left and/or right ends of the copolymer. For example, protection of the right end of the copolymer in Scheme 1 and deprotection of the left end permit the left end to be elaborated in further polymerization reactions. The polymerization reactions occurring at the left end can involve any kinds of monomers, can transpire any number of times, and can occur in any order and manner, as desired.

An initiator (e.g., the 1,6-hexanediol initiator in Scheme 1) can also initiate ROP as an unprotected diol, dithiol or diamine. In such a case, both the right and left ends of the polymer would be elaborated in the same way in the polymerization reactions.

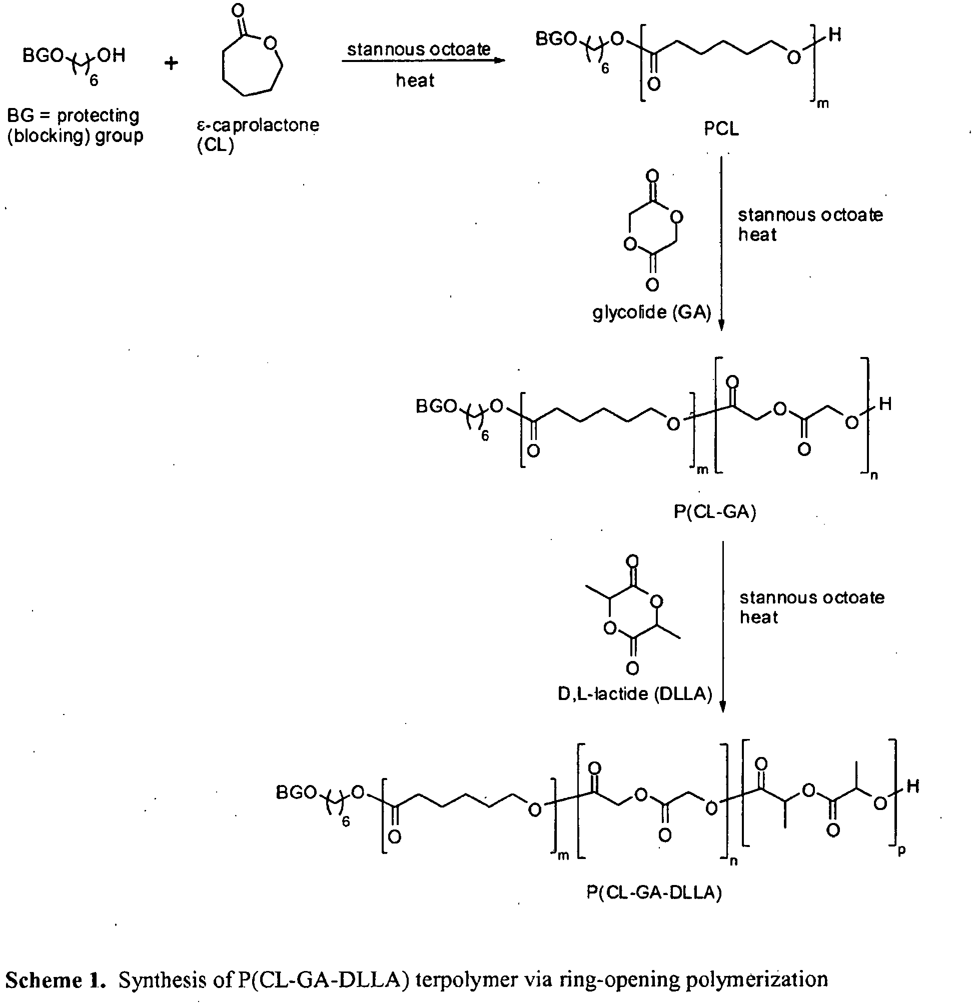

### Example 1. Controlled release of everolimus from P(DLLA-GA-CL) 60/15/25

A primer layer was formed on a stent from a primer solution containing about 2 weight % of P(DLLA-GA-CL) 60/15/25 (60 molar % DLLA, 15 molar % GA and 25 molar % CL) in 90/10 acetone/methyl isobutyl ketone (MIBK).

A solution containing about 2 weight % of P(DLLA-GA-CL) 60/15/25 and everolimus, at one of various drug-to-polymer (D:P) ratios, in 90/10 acetone/MIBK was prepared. The stent was mounted on a mandrel and spray-coated at a deposition rate of, e.g., about 10-20 microgram/pass. The stent was then dried in an oven at about 50 °C for about 30 minutes to evaporate the solvent. The dosage of everolimus was about 100 microgram/cm².

Drug release from the coated stent was analyzed by the "dipping" method. The stent was dipped in 10 mL of 0.1% sodium azide in porcine serum maintained at 37 ± 0.5 °C at a dipping rate of 40. The drug formulation was delivered into the release medium by a diffusion, dissolution and/or erosion process. Stent samples were withdrawn at 1- and 3-day intervals. At each time point, the stent was gently wiped with soft tissues and placed in 5 mL acetonitrile containing 0.02% BHT (butylated hydroxytoluene, specifically 2,6-bis-(t-butyl)-4-methylphenol). The stent was sonicated for 30 minutes to extract the drug. Samples (2 mL) were centrifuged at 13,000 rpm for 5 minutes for separation of any particulates, and analyzed by the optimized HPLC method. The amount of drug remaining on the stent after dipping was determined and the percent release of drug into the release medium was calculated using the assay/drug content values.

**Table 1** shows the release of everolimus from stents coated with P(DLLA-GA-CL) 60/15/25 at various conditions. The dosage of everolimus in all these examples was about 100 microgram/cm².

**Table 1. Release of everolimus from P(DLLA-GA-CL) 60/15/25**

| D:P Ratio | % Drug Release, Day 1 (n = 3) | % Drug Release, Day 3 (n = 3) |
|---|---|---|
| 1:3 | 66.7 ± 3.2 | 94.5 ± 0.7 |
| 1:5 | 56.5 ± 2.2 | 86.6 ± 0.5 |
| 1:3 with topcoat | 41.6 ± 2.6 | 77.4 ± 2.9 |

The results in Table I demonstrate that stents coated with P(DLLA-GA-CL) 60/15/25 terpolymers provide controlled release of everolimus and that the drug's release rate can be controlled by adjusting various factors such as, e.g., the D:P ratio and whether or not a topcoat layer is also applied.

### Example 2. Controlled release of everolimus from P(LLA-GA-CL) terpolymers

Similar procedures as those above were used to study the release of everolimus from drug-laden stents coated with P(LLA-GA-CL) terpolymers containing various molar percentages of LLA, GA and CL. **Figure 1** depicts the release of everolimus from stents coated with P(LLA-GA-CL) terpolymers, where the D:P ratio was 1:3 and the dosage of everolimus was about 100 microgram/cm². **Table 2** lists some of the results shown in Figure 1.

**Table 2. Release of everolimus from P(LLA-GA-CL) terpolymers, D:P = 1.3**

| Molar % of LLA/GA/CL | % Drug Release, Day 1 (n = 3) | % Drug Release, Day 3 (n = 3) |
|---|---|---|
| 40/30/30 | 88 ± 4.2 | 97.4 ± 1.5 |
| 50/25/25 | 58.6 ± 3 | 82.3 ± 2.3 |
| 60/15/25 | 47.4 ± 1.3 | 67.4 ± 4.5 |

As can be seen from Figure 1 and Table 2, stents coated with P(LLA-GA-CL) terpolymers provide controlled release of everolimus, and the drug's release rate can be controlled by adjusting various factor such as the molar % content of the LLA, GA and CL monomer components. Further, the results with P(DLLA-GA-CL) 60/15/25 and P(LLA-GA-CL) 60/15/25, both at a D:P ratio of 1:3, show that the release rate of a hydrophobic drug such as everolimus can also be controlled by the selection of the relatively polar monomer components - in this case, DLLA as compared to LLA.

While particular embodiments of the present invention have been shown and described, it will be obvious to those skilled in the art that changes and modifications can be made thereto without departing from the invention in its broader aspects. Therefore, the appended claims are to encompass within their scope all such changes and modifications as fall within the scope of the present invention.

It is understood that if a substance can have more than one stereochemistry and/or regiochemistry at one or more stereocenters and/or regiocenters and the stereochemistry and/or regiochemistry of the substance at the one or more stereocenters and/or regiocenters are not indicated, the scope of the present invention encompasses all possible stereoisomers (e.g., enantiomers, diastereomers, etc.) and/or regioisomers of that substance.

## Claims

1. An implantable device comprising a coating comprising a composition, wherein the coating has a thickness of ≤ 6 micron and completely degrades, which is the lost of at least 95 % of its mass, or substantially completely degrades, which is the lost of at least 75% of its mass, within 12 months when exposed to physiological conditions,
wherein the composition comprises a biodegradable block copolymer, wherein the copolymer:
is derived from at least two polar monomers selected from glycolide (GA), D-lactide (DLA), L-lactide (LLA), D,L-lactide (DLLA), and meso-lactide (MLA) and at least one nonpolar monomer selected from valerolactone (VL), caprolactone (CL), trimethylene carbonate (TMC), dioxanone (DS), hydroxybutyrate (HB), and hydroxyvalerate (HV);
has a T_{g} from -150 °C to 100°C;
has a polymer number-average molecular weight (Mₙ) from 10 kDa to 500 kDa; and
completely degrades, which is the lost of at least 95 % of its mass, or substantially completely degrades, which is the lost of at least 75% of its mass, within 12 months;
and wherein:
each kind of monomer independently has from 10 to 5,000 units
in the copolymer; and
the molar % of each kind of monomer independently is from 5% to 90%.

2. The implantable device comprising a coating of claim 1, wherein the biodegradable copolymer is selected from P(DLA-GA-VL), P(DLA-GA-CL), P(DLA-GA-TMC), P(DLA-GA-DS), P(DLA-GA-HB), P(DLA-GA-HV), P(LLA-GA-VL), P(LLA-GA-CL), P(LLA-GA-TMC), P(LLA-GA-DS), P(LLA-GA-HB), P(LLA-GA-HV), P(DLLA-GA-VL), P(DLLA-GA-CL), P(DLLA-GA-TMC), P(DLLA-GA-DS), P(DLLA-GA-HB), P(DLLA-GA-HV), P(MLA-GA-VL), P(MLA-GA-CL), P(MLA-GA-TMC), P(MLA-GA-DS), P(MLA-GA-HB), and P(MLA-GA-HV).

3. The implantable device comprising a coating of claim 1, which:
further comprises at least one biologically active agent selected from
antiproliferative, antineoplastic, antimitotic, anti-inflammatory, antiplatelet, anticoagulant, antifibrin, antithrombin, antibiotic, antiallergic and antioxidant substances; and
has one or a combination of a pulse, burst and sustained release profile for
each of the at least one biologically active agent.

4. The implantable device comprising a coating of claim 3, wherein the at least one biologically active agent is selected from paclitaxel, docetaxel, estradiol, dexamethasone, clobetasol, nitric oxide donors, super oxide dismutases, 4-amino-2,2,6,6-tetramethylpiperidirie-1-oxyl (4-amino-TEMPO), tacrolimus (FK-506), rapamycin (sirolimus), 40-*O*-(2-hydroxy)ethyl-rapamycin (everolimus), 40-*O*-(2-ethoxy)ethyl-rapamycin (biolimus), 40-*O*-(3-hydroxy)propyl-rapamycin, 40-*O*-[2-(2-hydroxy)ethoxy]ethyl-rapamycin, 40-*O*-tetrazole-rapamycin, 40-epi-(N1-tetrazolyl)-rapamycin (zotarolimus), pimecrolimus, imatinib mesylate, midostaurin, progenitor cell-capturing antibodies, and combinations thereof.

5. The implantable device comprising a coating of claim 3, wherein the bioactive agent-to-copolymer mass ratio for each of the at least one bioactive agent independently is from 1:1 to 1:10.

6. The implantable device comprising a coating of claim 3, wherein the at least one biologically active agent has a sustained release over a period up to 12 months, wherein the sustained release is determined according to the method in the description.

7. The implantable device comprising a coating of claim 6, wherein the at least one biologically active agent has a sustained release over a period up to 3 months, wherein the sustained release is determined according to the method in the description.

8. The device of claim 1, which is selected from stents, grafts, stent-grafts, catheters, leads, electrodes, clips, shunts, closure devices, valves, and particles.

9. An implantable device formed of a material comprising the coating as defined in claim 1, for use in the treatment or prevention of a condition or disorder in a patient selected from the group consisting of atherosclerosis, thrombosis, restenosis, hemorrhage, vascular dissection, vascular perforation, vascular aneurysm, vulnerable plaque, chronic total occlusion, patent foramen ovale, claudication, anastomotic proliferation of vein and artificial grafts, arteriovenous anastamoses, bile duct obstruction, and tumor obstruction.

## Patentansprüche

1. Implantierbare Vorrichtung umfassend eine Beschichtung umfassend eine Zusammensetzung, wobei die Beschichtung eine Dicke von ≤ 6 Mikrometer hat und innerhalb 12 Monate, wenn sie physiologischen Bedingungen ausgesetzt wird, vollständig abgebaut wird, was die Abnahme von mindestens 95% ihrer Masse ist, oder im Wesentlichen vollständig abgebaut wird, was die Abnahme von mindestens 75% ihrer Masse ist,
wobei die Zusammensetzung ein biologisch abbaubares Block-Copolymer umfasst, wobei das Copolymer:
von mindestens zwei polaren Monomeren, die ausgewählt sind aus Glycolid (GA), D-Lactid (DLA), L-Lactid (LLA), D,L-Lactid (DLLA), und Meso-Lactid (MLA) und
mindestens einem nicht polaren Monomer, das ausgewählt ist aus Valerolacton (VL),
Caprolacton (CL), Trimethylencarbonat (TMC), Dioxanon (DS), Hydroxybutyrat (HB),
und Hydroxyvalerat (HV), abgeleitet ist;
eine Tg von -150 °C bis 100 °C hat;
ein Zahlenmittel des Molekulargewichts (Mₙ) des Polymeren von 10 kDa bis 500 kDa hat; und
innerhalb 12 Monate vollständig abgebaut wird, was die Abnahme von mindestens 95% seiner Masse ist, oder im Wesentlichen vollständig abgebaut wird, was die Abnahme von mindestens 75% seiner Masse ist, und wobei:
jede Art der Monomere unabhängig voneinander von 10 bis 5.000 Monomerbausteine im Copolymer hat; und
das Mol % von jeder Art der Monomere unabhängig voneinander von 5% bis 90% ist.

2. Implantierbare Vorrichtung umfassend eine Beschichtung nach Anspruch 1, wobei das biologisch abbaubare Copolymer ausgewählt aus P(DLA-GA-VL), P(DLAGA-CL), P(DLA-GA-TMC), P(DLA-GA-DS), P(DLA-GA-HB), P(DLA-GA-HV), P(LLA-GA-VL), P(LLA-GA-CL), P(LLA-GA-TMC), P(LLA-GA-DS), P(LLA-GA-HB), P(LLA-GA-HV), P(DLLA-GA-VL), P(DLLA-GA-CL), P(DLLA-GA-TMC), P(DLLA-GA-DS), P(DLLA-GA-HB), P(DLLA-GA-HV), P(MLA-GA-VL), P(MLA-GA-CL), P(MLA-GA-TMC), P(MLA-GA-DS), P(MLA-GA-HB), und P(MLA-GA-HV) ist.

3. Implantierbare Vorrichtung umfassend eine Beschichtung nach Anspruch 1, welche:
weiterhin mindestens einen biologischen Wirkstoff umfasst, der ausgewählt aus antiproliferativen, antineoplastischen, antimitotischen, entzündungshemmenden, thrombozyten-aggregationshemmenden, gerinnungshemmenden, antifibrinen, antithrombinen, antibiotischen, antiallergischen und antioxidativen Substanzen ist; und
für jeden der mindestens einen biologischen Wirkstoff ein Freisetzungsprofil hat, das eins von Puls, Ausbruch und anhaltender Freisetzung oder eine Kombination davon ist.

4. Implantierbare Vorrichtung umfassend eine Beschichtung nach Anspruch 3, wobei der mindestens eine biologische Wirkstoff ausgewählt ist aus Paclitaxel, Docetaxel, Estradiol, Dexamethason, Clobetasol, Stickstoffmonoxiddonatoren, Superoxid-Dismutasen, 4-Amino-2,2,6,6-tetramethylpiperidin-1-oxyl (4-Amino-TEMPO), Tacrolimus (FK-506), Rapamycin (Sirolimus), 40-O-(2-Hydroxy)ethyl-rapamycin (Everolimus), 40-O-(2-Ethoxy)ethyl-rapamycin (Biolimus), 40-O-(3-Hydroxy)propyl-rapamycin, 40-O-[2-(2-Hydroxy)ethoxy]ethyl-rapamycin, 40-O-Tetrazol-rapamycin, 40-Epi-(N1-tetrazolyl)-rapamycin (Zotarolimus), Pimecrolimus, Imatinibmesylat, Midostaurin, Antikörpern, die Vorläuferzellen einfangen und Kombinationen davon.

5. Implantierbare Vorrichtung umfassend eine Beschichtung nach Anspruch 3, wobei das Gewichtsverhältnis vom biologischen Wirkstoff zum Copolymer für jeden der mindestens einen biologischen Wirkstoff unabhängig voneinander von 1:1 bis 1:10 reicht.

6. Implantierbare Vorrichtung umfassend eine Beschichtung nach Anspruch 3, wobei der mindestens eine biologische Wirkstoff eine anhaltende Freisetzung über einen Zeitraum von bis 12 Monate hat, wobei die anhaltende Freisetzung nach dem Verfahren der Beschreibung ermittelt wird.

7. Implantierbare Vorrichtung umfassend eine Beschichtung nach Anspruch 6, wobei der mindestens eine biologische Wirkstoff eine anhaltende Freisetzung über einen Zeitraum von bis 3 Monate hat, wobei die anhaltende Freisetzung nach dem Verfahren der Beschreibung ermittelt wird.

8. Vorrichtung nach Anspruch 1, die ausgewählt aus Stents, Propfen, Stent-Propfen, Kathetern, Leads, Elektroden, Klammern, Shunts, Verschlussvorrichtungen, Ventilen, und Partikeln ist.

9. Implantierbare Vorrichtung, die aus einem Material umfassend die Beschichtung wie im Anspruch 1 definiert gebildet ist zur Verwendung bei der Behandlung oder Vorbeugung eines Zustands oder einer Störung bei einem Patienten, der/die ausgewählt ist aus der Gruppe bestehend aus Atherosklerose, Thrombose, Restenose, Hämorrhagien, Gefäßdissektion, Gefäßperforation, Gefäßaneurysma, vulnerablen Plaques, chronischem Totalverschluss, offenem Foramen ovale, Klaudication, anastomotischer Proliferation eines Gefäßes und künstlichen Implantaten, arteriovenösen Anastomosen, Gallengänge-Obstruktion, und tumorbedingten Obstruktion.

## Revendications

1. Dispositif implantable comprenant une couche comprenant une composition, dans lequel la couche a un épaisseur de ≤ 6 micromètres et se dégrade complètement, ce qui est la perte d'au moins 95% de sa masse, ou elle se dégrade de façon essentiellement complète, ce qui est la perte d'au moins 75% de sa masse, dans 12 mois, lorsqu'elle est exposée à des conditions physiologiques,
dans lequel la composition comprend un copolymère bloc biodégradable,
dans lequel le copolymère:
est dérivé d'au moins deux monomères polaires choisis de glycolide (GA), D-lactide (DLA), L-lactide (LLA), D,L-lactide (DLLA), et méso-lactide (MLA) et au moins un monomère non-polaire choisi parmi la valérolactone (VL), la caprolactone (CL), le carbonate de triméthylène (TMC), la dioxanone (DS), l'hydroxybutyrate (HB), et
l'hydroxyvalérate (HV);
a une Tg de -150 °C à 100°C;
a un poids moléculaire de polymère moyen en nombre (Mₙ) de 10 kDa à 500 kDa; et
il se dégrade complètement, ce qui est la perte d'au moins 95% de sa masse, ou il se dégrade de façon essentiellement complète, ce qui est la perte d'au moins 75% de sa masse, dans 12 mois; et dans lequel:
chaque sorte de monomère indépendamment a de 10 à 5.000 unités dans le copolymère; et
le % molaire de chaque sorte de monomère indépendamment est de 5% à 90%.

2. Le dispositif implantable comprenant une couche de la revendication 1, dans lequel le copolymère biodégradable est choisi parmi P(DLA-GA-VL), P(DLAGA-CL), P(DLA-GA-TMC), P(DLA-GA-DS), P(DLA-GA-HB), P(DLA-GA-HV), P(LLA-GA- VL), P(LLA-GA-CL), P(LLA-GA-TMC), P(LLA-GA-DS), P(LLA-GA-HB), P(LLA-GA-HV), P(DLLA-GA-VL), P(DLLA-GA-CL), P(DLLA-GA-TMC), P(DLLA-GA-DS), P(DLLA-GA-HB), P(DLLA-GA-HV), P(MLA-GA-VL), P(MLA-GA-CL), P(MLA-GA- TMC), P(MLA-GA-DS), P(MLA-GA-HB), et P(MLA-GA-HV).

3. Le dispositif implantable comprenant une couche de la revendication 1, qui:
comprend en outre au moins un agent biologiquement actif choisi parmi les substances antiprolifératives, antinéoplasiques, antimitotiques, anti-inflammatoires, antiplaquettaires, anticoagulantes, antifibrines, antithrombines, antibiotiques,
antiallergiques et antioxydantes; et qui
a une ou une combinaison de libération pulsée, déchaînement et libération prolongée pour chacun de l'au moins un agent biologiquement actif.

4. Le dispositif implantable comprenant une couche de la revendication 3, dans lequel l'au moins un agent biologiquement actif est choisi parmi le paclitaxel, le docétaxel, l'oestradiol, la déxaméthasone, le clobétasol, les donneurs d'oxyde nitrique, les super-oxyde-dismutases, le 4-amino-2,2,6,6-tetraméthylpipéridine-1-oxyl (4-amino-TEMPO), le tacrolimus (FK-506), la rapamycine (sirolimus), la 40-O-(2-hydroxy)éthyl-rapamycine (évérolimus), la 40-O-(2-éthoxy)éthyl-rapamycine (biolimus), la 40-O-(3-hydroxy)propyl-rapamycine, la 40-O-[2-(2-hydroxy)éthoxy]éthyl-rapamycine, la 40-O-tétrazol-rapamycine, la 40-epi-(N1-tétrazolyl)-rapamycine (zotarolimus), le pimécrolimus, le mésylate d'imatinib, la midostaurine, les anticorps de capture de cellules progénetrices et des combinaisons de ceux-ci.

5. Le dispositif implantable comprenant une couche de la revendication 3, dans lequel le rapport de masse de l'agent bioactif au copolymère pour chacun de l'au moins un agent bioactif indépendamment est de 1:1 à 1:10.

6. Le dispositif implantable comprenant une couche de la revendication 3, dans lequel l'au moins un agent biologiquement actif a une libération prolongée pendant une période de jusqu'à 12 mois, dans lequel la libération prolongée est déterminée selon la méthode de la description.

7. Le dispositif implantable comprenant une couche de la revendication 6, dans lequel l'au moins un agent biologiquement actif a une libération prolongée pendant une période de jusqu'à 3 mois, dans lequel la libération prolongée est déterminée selon la méthode de la description.

8. Le dispositif de la revendication 1, qui est choisi parmi des endoprothèses vasculaires, des greffes, des endoprothèses vasculaires-greffes, des cathéters, des liens, des électrodes, des clips, des shunts, des dispositifs de fermeture, des valvules, et des particules.

9. Un dispositif implantable formé d'un matériau comprenant la couche tel que définie dans la revendication 1, pour l'utilisation dans le traitement ou prévention dans un patient d'un état ou trouble choisi(e) dans le groupe constitué de l'athérosclérose, la thrombose, la resténose, les hémorrhagies, la dissection vasculaire, la perforation vasculaire, l'anéurisme vasculaire, la plaque vulnérable, l'occlusion totale cronique, le foramen ovale perméable, la claudication, la prolifération anastomotique des veines et des greffes artificielles, les anastomoses artérioveineuses, l'obstruction du canal biliaire, et l'obstruction tumorale.
